# EUROPEAN PATENT APPLICATION

(11) **EP 3 181 569 A1**
(43) Date of publication of application: **21.06.2017**
(21) Application number: 15831628.1
(22) Date of filing: 10.08.2015
(51) Int. Cl.: C07D 519/00, C09D 11/03, C09K 11/06, H01L 29/786, H01L 51/05, H01L 51/30, H01L 51/46, H01L 51/50

(54) **BENZOBIS(THIADIAZOLE) DERIVATIVE, INK CONTAINING SAME, AND ORGANIC ELECTRONIC DEVICE USING SAME**

(30) Priority: 13.08.2014 JP 2014164975
(71) Applicant: UBE Industries, Ltd., Ube-shi, Yamaguchi 755-8633 (JP); National University Corporation Yamagata University, Yamagata 990-8560 (JP)
(72) Inventor: TOKITO, Shizuo, Yonezawa-shi Yamagata 992-8510 (JP); KUMAKI, Daisuke, Yonezawa-shi Yamagata 992-8510 (JP); MAMADA, Masashi, Fukuoka-shi Fukuoka 812-8581 (JP); HONMA, Takashi, Ichihara-shi Chiba 290-0045 (JP); TANAKA, Yasuhiro, Ichihara-shi Chiba 290-0045 (JP); MACHIDA, Toshikazu, Ichihara-shi Chiba 290-0045 (JP); KAKITA, Kazuaki, Ichihara-shi Chiba 290-0045 (JP); YAMADA, Natsuko, Ichihara-shi Chiba 290-0045 (JP)
(74) Representative: Hoffmann Eitle
(86) International application number: PCT/JP2015/072644
(87) International publication number: WO 2016/024567

(57) **Abstract**

An object of the present invention is to provide a benzobis(thiadiazole) derivative, which has an excellent mobility of electron (field-effect mobility) and also has an excellent stability in the atmosphere. The present invention relates to a benzobis(thiadiazole) derivative or the like, which has cyclic imide structures annelated to an aromatic ring in the molecule, represented by the following general formula (1) or (2) wherein R, A and Z represent predetermined groups.

## Description

### Technical Field

The present invention relates to a benzobis(thiadiazole) derivative, an organic semiconductor ink comprising the same, and organic electronic devices including an organic thin film transistor, an organic electroluminescence device, a display device, an organic thin film photovoltaic cell, a RFID tag, and a sensor using the same.

### Background Art

Conventionally, benzobisthiazole compounds attract attention as a compound used for an organic thin film transistor (organic TFT), an organic electroluminescence device (organic EL device) or an organic thin film photovoltaic cell. Thus, various derivatives in which the main skeleton is benzobis(thiadiazole) are studied and developed vigorously.

A benzobis(thiadiazole) derivative into which a strong electron-withdrawing group is introduced in order to improve the mobility of hole and electron, or the stability in the atmosphere, in particular, is proposed. For example, Patent Document 1 as well as Non Patent Document 1 and Non Patent Document 2 disclose a compound in which trifluoromethylphenyl group or the like is bound to benzobis(thiadiazole) via thienylene group. This compound has a mobility of hole and electron improved by the introduction of trifluoromethylphenyl group or the like which is a strong electron-withdrawing group.

Patent Document 2 also discloses various benzobisthiadiazole compounds as an n-type organic semiconductor material. However, the compound practically synthesized in the example of such document is 4,8-bis[3,5-bis(trifluoromethyl)phenyl] benzo[1,2-c;4,5-c']bis[1,2,5]thiadiazole (see the following structure) only.

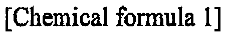

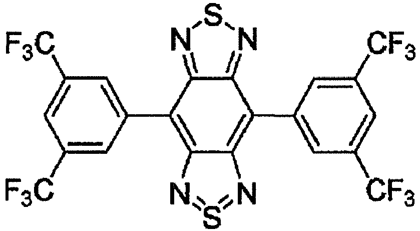

Meanwhile, it is generally known that a compound having a strong electron-withdrawing group introduced into thiophene ring has an improved stability or mobility of electron, although the compound is not a compound in which the main skeleton is benzobis(thiadiazole) (See, for example, Patent Document 3).

In Non Patent Document 3 and Non Patent Document 4, compounds in which a substituent of a thienobisimide structure is bound to a bithiazole skeleton, thienothiophene skeleton, bithiophene skeleton and quarterthiophene skeleton were synthesized. The same documents reported that these compounds exhibited the transistor characteristics.

### Prior art Documents

### Patent Documents

Patent Document 1: WO2013/141182 Brochure
Patent Document 2: JP-A-2013-124231
Patent Document 3: JP-A-2009-280515

### Non Patent Documents

Non Patent Document 1: Chem.Commun., 46, 3265 (2010)
Non Patent Document 2: Applied Physics Lett., 97, 133303 (2010)
Non Patent Document 3: Chem.Commun., 49, 4298 (2013)
Non Patent Document 4: Chem.Mater. 25, 668 (2013)

### Summary of Invention

### Problems to be solved by the invention

Patent Document 2 discloses various benzobisthiadiazole compounds as an n-type organic semiconductor material. As described above, however, the compound practically synthesized is 4,8-bis[3,5-bis(trifluoromethyl)phenyl]benzo[1,2-c;4,5-c']bis[1,2,5]thiadiazole only. Additionally, although the result of the measurement of cyclic voltammetry (CV) of the compound is described in Patent Document 2, a device such as a thin film transistor (TFT) was not produced with the compound and was not evaluated for the characteristics. Thus, in Patent Document 2, it has not been confirmed whether the compound practically synthesized in the example has sufficient characteristics as an organic semiconductor material.

Non Patent Document 3 and Non Patent Document 4 describe that the compound in which a substituent of a thienobisimide structure is bound to various skeletons exhibits both p-type and n-type characteristics. It is known that the both characteristics are the unfavorable characteristics as a transistor device. The position of LUMO energy level of the center skeleton structure bound to the thienobisimide structure is considered to be the cause for the both characteristics. It is very difficult to significantly change the LUMO energy level of the center skeleton structure only by changing the substituent on the thienobisimide structure. Thus, in order to obtain organic semiconductor materials exhibiting p-type or n-type characteristic only, there is a need to fundamentally change the molecular design thereof.

An object of the present invention is to provide a benzobis(thiadiazole) derivative, which has an excellent mobility of electron (field-effect mobility) and also has an excellent stability in the atmosphere. Desirably, an object of the present invention is to provide a benzobis(thiadiazole) derivative which dominantly exhibits the characteristics of any one of a p-type or n-type. Further, an object of the present invention is to provide organic electronic devices including an organic thin film transistor, an organic electroluminescence device, a display device, an organic thin film photovoltaic cell, a RFID tag, and a sensor using these benzobis(thiadiazole) derivatives.

### Means for solving the problem

The present invention relates to the following items.
1. A benzobis(thiadiazole) derivative, which has cyclic imide structures annelated to an aromatic ring in the molecule, represented by the following general formula (1) or (2): 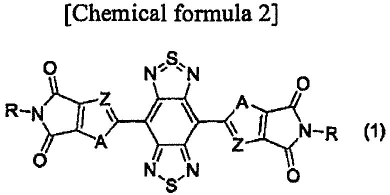 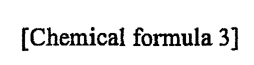 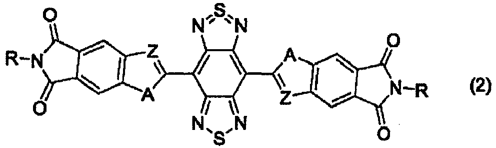 Wherein the above formulae (1) and (2),
   two Rs represent independently a linear or branched alkyl group, or a linear or branched arylalkyl group, wherein a hydrogen atom in the alkyl group and in the alkyl group in the arylalkyl group can be substituted by a fluorine atom,
   two As represent independently an oxygen atom, a sulfur atom or a selenium atom, and
   two Zs represent independently a methine carbon or a nitrogen atom.
2. The benzobis(thiadiazole) derivative as described in 1, wherein two As are a sulfur atom, two Zs are independently a methine carbon or a nitrogen atom, and two Rs are independently a linear or branched alkyl group.
3. The benzobis(thiadiazole) derivative as described in 1 or 2, wherein two Rs are a linear or branched alkyl group of 5 to 25 carbon atoms.
4. The benzobis(thiadiazole) derivative as described in any one of 1 to 3, wherein the benzobis(thiadiazole) derivative is a compound represented by the general formula (1).
5. The benzobis(thiadiazole) derivative as described in any one of 1 to 4, wherein the benzobis(thiadiazole) derivative is soluble in an organic solvent.
6. An organic semiconductor ink comprising the benzobis(thiadiazole) derivative as described in any one of 1 to 5.
7. An organic semiconductor ink comprising two or more of organic semiconductors, wherein one or more of the organic semiconductors are the benzobis(thiadiazole) derivative as described in any one of 1 to 5.
8. An organic electronic device comprising an organic layer, which comprises the benzobis(thiadiazole) derivative as described in any one of 1 to 5.
9. An organic thin film transistor, comprising a gate electrode, a gate insulating layer, an organic semiconductor layer, a source electrode and a drain electrode on a substrate, wherein the organic semiconductor layer comprises the benzobis(thiadiazole) derivative as described in any one of 1 to 5.
10. The organic thin film transistor as described in 9, wherein the substrate is a flexible substrate.
11. An organic electroluminescence device comprising an anode, a luminescent layer, a hole transport layer and/or an electron transport layer, and a cathode on a substrate, wherein the hole transport layer and/or the electron transport layer comprises the benzobis(thiadiazole) derivative as described in any one of 1 to 5.
12. The organic electroluminescence device as described in 11, wherein the substrate is a flexible substrate.
13. A display device, in which an organic electroluminescence device is driven/lighted by the use of an organic thin film transistor, wherein the organic thin film transistor is the organic thin film transistor as described in 9 or 10.
14. An active-matrix display device, wherein pixels are arranged in a matrix form, the pixel comprising the organic thin film transistor as described in 9 or 10 and an organic electroluminescence device.
15. The display device as described in 13 or 14, wherein the organic electroluminescence device is the organic electroluminescence device as described in 11 or 12.
16. A display device, in which an organic electroluminescence device is driven/lighted by the use of an organic thin film transistor, wherein the organic electroluminescence device is the organic electroluminescence device as described in 11 or 12.
17. An organic thin film photovoltaic cell comprising a anode, a charge separation layer comprising a hole transport material and an electron transport material, and an cathode on a substrate, wherein the charge separation layer comprises the benzobis(thiadiazole) derivative as described in any one of 1 to 5.
18. An organic thin film photovoltaic cell comprising an anode, a charge separation layer comprising a hole transport material and an electron transport material, a hole transport layer and/or an electron transport layer, and a cathode on a substrate, wherein the hole transport layer and/or the electron transport layer comprises the benzobis(thiadiazole) derivative as described in any one of 1 to 5.
19. The organic thin film photovoltaic cell as described in 17 or 18, wherein the substrate is a flexible substrate.
20. A RFID tag, which is activated by the use of an organic thin film transistor, wherein the organic thin film transistor is the organic thin film transistor as described in 9 or 10.
21. A sensor, which is activated by the use of an organic thin film transistor, wherein the organic thin film transistor is the organic thin film transistor as described in 9 or 10.

### Advantageous Effects of Invention

According to the present invention, there may be provided a benzobis(thiadiazole) derivative which has an excellent mobility of electron (field-effect mobility), and also has an excellent stability in the atmosphere. The benzobis(thiadiazole) derivative of the present invention has an excellent mobility of electron (field-effect mobility), and also has an excellent stability in the atmosphere. Thus, the derivatives may be suitably used for, for example, organic electronic devices including an organic thin film transistor, an organic electroluminescence device, a display device, a display, an organic thin film photovoltaic cell, a RFID tag, and a sensor. The compound may be also suitably used for many other devices. Additionally, the benzobis(thiadiazole) derivative of the present invention desirably dominantly exhibits the semiconductor characteristics of any one of p-type or n-type.

### Brief Description of Drawings

Fig. 1 is a sectional view illustrating an example of the layer configuration of the organic thin film transistor (organic TFT) of the present invention.
Fig.2 is a diagram illustrating one example of the complementary inverter (logic inverting) circuit.
Fig.3 is a sectional view illustrating the layer configuration of one example of the organic electroluminescence device (organic EL device) of the present invention.
Fig.4 is a sectional view illustrating the layer configuration of one example of the display device of the present invention.
Fig.5 is a sectional view illustrating the layer configuration of one example of the organic thin film photovoltaic cell of the present invention.
Fig.6 is a graph showing the transfer characteristic of the organic TFT of Example E-1a.
Fig.7 is a graph showing the transfer characteristic of the organic TFT of Example E-1e.

### Mode for carrying out the invention

The benzobis(thiadiazole) derivative, the organic semiconductor ink, and the organic electronic devices of the present invention are described below in detail.

### <Benzobis(thiadiazole) derivative>

The benzobis(thiadiazole) derivative of the present invention is represented by the following general formula (1) or (2).

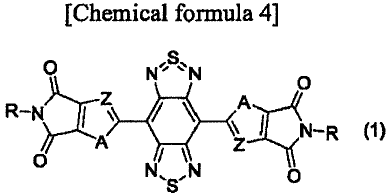

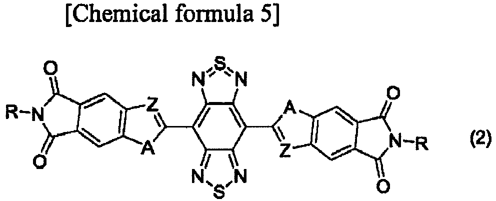

As shown from the structure represented by the general formulae (1) and (2), the benzobis(thiadiazole) derivative of the present invention is a compound which has cyclic imide structures annelated to an aromatic ring in the molecule. R, A and Z groups in these general formulae are described below in turn.

### (For R group)

In the general formulae (1) and (2), R represents a linear or branched alkyl group, or a linear or branched arylalkyl group. A hydrogen atom in the alkyl group and in the alkyl group in the arylalkyl group can be substituted by a fluorine atom. There are two Rs in the general formulae (1) and (2) respectively, and these Rs are independent of each other and may be same or different.

For the linear or branched alkyl group, from the viewpoint of the improvement in field-effect mobility and solubility of the benzobis(thiadiazole) derivative of the present invention, the carbon number of the alkyl group is preferably 1 to 30, more preferably 3 to 28, even more preferably 5 to 25, and particularly preferably 5 to 15. Also, for the branched alkyl group, the branched chain moiety and the main chain moiety may be bounded each other to form the cyclic structure. That is, the branched alkyl groups include a cycloalkyl group.

Specific examples of the linear alkyl group include, for example, methyl group, ethyl group, propyl group, butyl group, pentyl group, hexyl group, heptyl group, octyl group, nonyl group, decyl group, undecyl group, dodecyl group, tridecyl group, tetradecyl group, pentadecyl group, hexadecyl group, heptadecyl group, and octadecyl group, and the following structures also are included in the examples (but some of which overlap with those exemplified above). A hydrogen atom on these alkyl groups can be substituted by a fluorine atom.

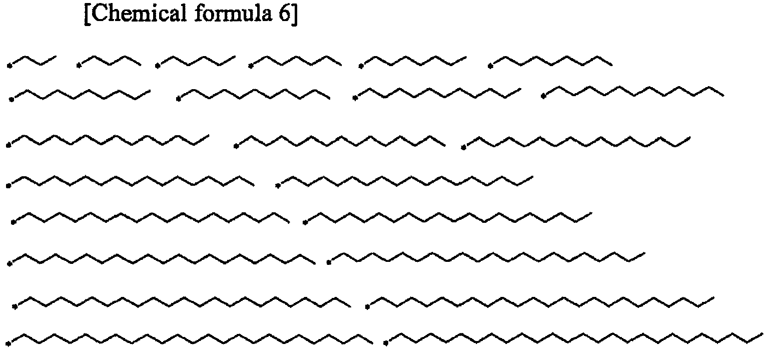

In the structures as described above, an asterisk (*) denotes a bond. The same shall apply hereinafter.

Specific examples of the branched alkyl group include, for example, isopropyl group, 1-methylpropyl group, 1-methylbutyl group, 2-methylhexyl group, 2-ethylhexyl group, 3-methylhexyl group, 3-ethylhexyl group, 2-methyloctyl group, 2-ethyloctyl group, 2-hexyldecyl group, 2-octyldodecyl group, 2-decyltetradecyl group, 3-methyloctyl group, and 3-ethyloctyl group, and the following structures also are included in the examples (but some of which overlap with those exemplified above). A hydrogen atom on these alkyl groups can be substituted by a fluorine atom.

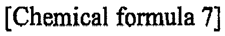

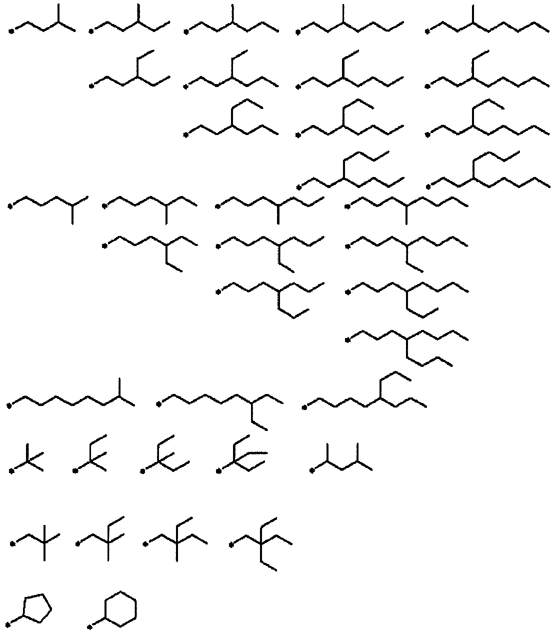

Among the alkyl group as described above, alkyl groups having a branched structure, which has not a branched chain on a carbon directly linked to a nitrogen atom, is preferred. Suitable examples of such alkyl group include 2-methylhexyl group, 2-ethylhexyl group, 3-methylhexyl group, 3-ethylhexyl group, 2-methyloctyl group, 2-ethyloctyl group, 2-hexyldecyl group, 2-octyldodecyl group, 2-decyltetradecyl group, 3-methyloctyl group, 3-ethyloctyl group and the like. Also, in the general formula (1), if A is a sulfur atom and Z is a methine carbon, R is preferably not 1-methylhexyl group.

For the linear or branched arylalkyl group as described above, the term "linear or branched" as used herein means that a structure of an alkyl group moiety in an arylalkyl group is linear or branched. Also, in addition to an alkyl group linked to an N atom in the general formulae (1) and (2), an aryl moiety in an arylalkyl group may be linked to one or more alkyl groups.

Examples of the linear arylalkyl group as described above include benzyl group, 2-phenylethyl group, 1-naphthyl methyl group, 2-naphthyl-2-ethyl group and the like.

Examples of the branched arylalkyl group include diphenylmethyl group, triphenylmethyl group, 1,2-diphenylethyl group, 4-phenylhexyl group, 1-phenylethyl group, 1-(4-methylphenyl)ethyl group and the like.

### (For A group)

In the general formulae (1) and (2) as described above, A group represents an oxygen atom, a sulfur atom or a selenium atom. There are two A groups in the general formulae (1) and (2), and these A groups are independent of each other and may be same or different. From the viewpoint of the stability in the atmosphere of the benzobis(thiadiazole) derivative of the present invention, A group is preferably a sulfur atom.

### (For Z group)

In the general formulae (1) and (2) as described above, Z group represents a methine carbon (CH) or a nitrogen atom. There are two Z groups in the general formulae (1) and (2), and these Z groups are independent of each other and may be same or different. From the viewpoint of the stability in the atmosphere of the benzobis(thiadiazole) derivative of the present invention, Z group is preferably any one of a methine carbon and a nitrogen atom, but from the viewpoint of the raw materials availability, Z group is preferably a methine carbon.

### (Preferred combination of R, A and Z groups)

In the general formulae (1) and (2) as described above, a preferred combination of R, Z and A groups is as follows. That is, in the preferred embodiment of the present invention, from the viewpoint of the stability in the atmosphere and raw materials availability of the benzobis(thiadiazole) derivative of the present invention, it is preferred that A group is a sulfur atom, Z is independently a methine carbon or a nitrogen atom, and R is independently a linear or branched alkyl group in the general formulae (1) and (2).

As mentioned above, in the general formulae (1) and (2), two A groups, two Z groups and two R groups all may be same or different, but are preferably the same from the viewpoint of the synthetic accessibility of the benzobis(thiadiazole) derivative of the present invention.

The benzobis(thiadiazole) derivative of the present invention is the compound of the general formula (1) or (2), and preferably the compound of the general formula (1) from the viewpoint of the field-effect mobility.

### (Benzobis(thiadiazole) derivative)

The benzobis(thiadiazole) derivative of the present invention as described above include, for example, compounds represented by formulae (11) to (15) and (21) to (25) wherein, R is as defined above.

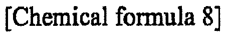

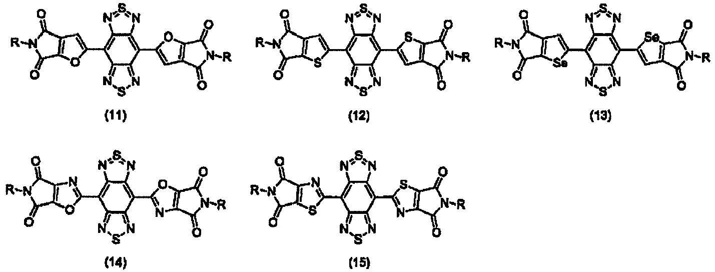

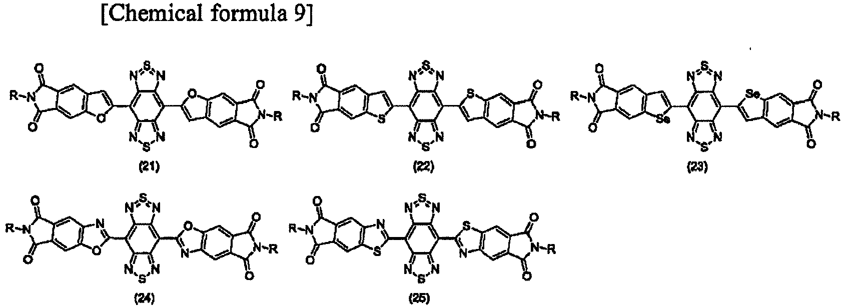

### (Synthesis method of benzobis(thiadiazole) derivative)

The benzobis(thiadiazole) derivative of the present invention can be synthesized by combinating any known raw materials and reactions. For example, according to the reaction scheme described below, the derivatives can be synthesized.

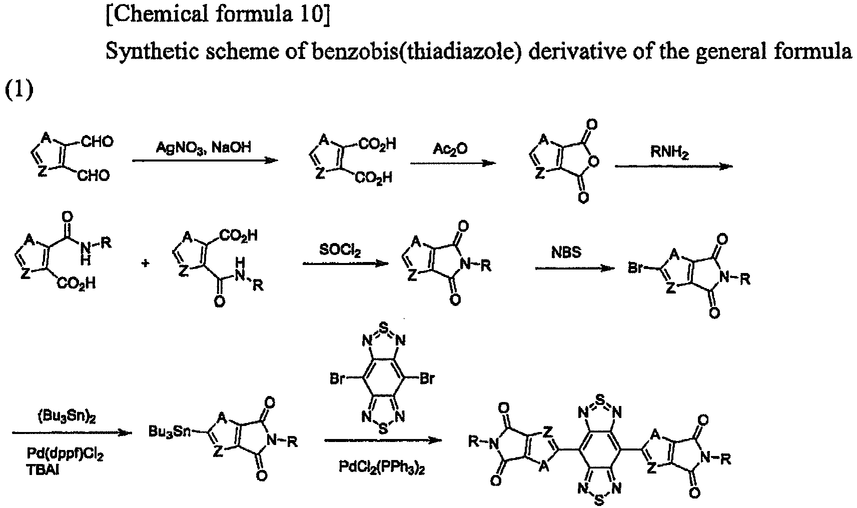

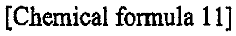

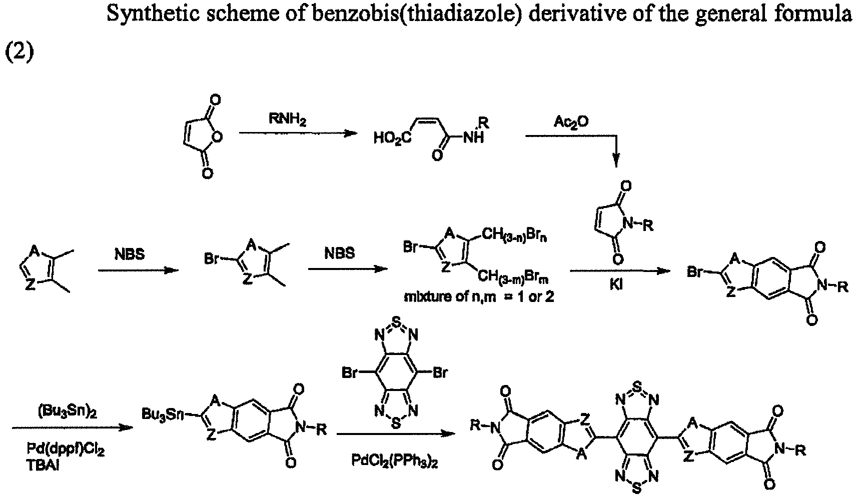

In the synthetic scheme described above, R, A and Z groups are as defined above. Also, Bu represents butyl group.

For synthetic scheme of benzobis(thiadiazole) derivative of the general formula (1), a dialdehyde compound having A and Z groups as a ring constituting atom is first prepared. The compound is commercially available, or may be synthesized by any known methods.

The dialdehyde compound is further oxidized to an anhydride. This is reacted with an amine having R group (RNH₂) to form an amide group while ring opening the anhydride group. This is reacted with chlorinating agents such as thionyl chloride to activate a carbonyl group, thereby causing the ring closing again to form an imide structure (R is liked to an N atom). In addition to thionyl chloride, for example, zinc halide and acetic anhydride may also be used.

This is reacted with N-bromosuccinimide (NBS) to introduce a bromo atom at a predetermined position. Subsequently, the resulting product is reacted with a predetermined organostannic reagent in the presence of a palladium catalyst (1,1'-bis(diphenylphosphino)ferrocene palladium (II) dichloride-dichloromethane complex in this scheme) and tetrabutylammonium iodide (TBAI) to organostannic the position in which the bromo atom is introduced in the product.

The organostannic compound thus obtained is reacted with a benzobis(thiadiazole) in which a bromo atom is introduced in the benzene ring position to obtain the benzobis(thiadiazole) derivative of the general formula (1) (Stille coupling. The benzobis(thiadiazole) in which the bromo atom is introduced are commercially available, or may be synthesized by any known raw materials and reactions). Rather than the organostannic compound, the product in which the bromo atom is introduced may be reacted with a zinc reagent to a zinc compound, which reacted with the benzobis(thiadiazole) in which the bromo atom is introduced to obtained the benzobis(thiadiazole) derivative of the general formula (1) (Negishi coupling).

Then, for synthetic scheme of the benzobis(thiadiazole) derivative of the general formula (2), a maleic anhydride is first reacted with an amine having R group (RNH₂) to ring open to form an amide group. This is ring closed to form an imide for example in the presence of acetic anhydride.

On the other hand, a dimethyl compound having A and Z groups as a ring constituting atom is prepared. The compound is commercially available, or may be synthesized by any known methods. The compound is reacted with NBS to introduce a bromo atom at a predetermined position or methyl group in the ring.

The compound thus obtained is reacted with the obtained imide in the presence of potassium iodide to fuse these two five-membered rings such that the benzene ring is formed between these at the annelated state. The bromo atom linked to the ring having A and Z groups as a ring constituting atom is remained.

The annelated cyclic compound thus obtained is reacted with a predetermined organostannic reagent in the presence of a palladium catalyst and TBAI to organostannic the position in which the bromo atom is introduced.

The organostannic compound thus obtained is reacted with the benzobis(thiadiazole) in which the bromo atom is introduced at the benzene ring position to obtained the benzobis(thiadiazole) derivative of the general formula (2) (Stille coupling).

Palladium catalysts used in the synthesis of the benzobis(thiadiazole) derivative of the present invention as described above include, in addition to those as described in the synthetic scheme, one or two or more of palladium complexes such as tetrakis(triphenylphosphine) palladium (Pd(PPh₃)₄), bis(tri-tert-butylphosphine) palladium (Pd(PtBu₃)₂), tris(dibenzylideneacetone) palladium (Pd₂(dba)₃), palladium acetate (Pd(OAc)₂) and palladium chloride (PdCl₂) may be used. The amount of the palladium catalyst is preferably used at 0.01 to 0.5 mol, more preferably at 0.1 to 0.5 mol per 1 mol of the benzobis(thiadiazole) in which the bromo atom is introduced at the benzene ring position.

Moreover, bases, halogenating reagents or the like which may be used are not limited the reagents in the synthetic scheme as described above, and those conventionally used in the reaction as described above may be used without limiting.

After the completion of the reaction of the synthetic scheme as described above, the benzobis(thiadiazole) derivative of the present invention may be isolated and purified from the obtained reaction solution by performing common operations such as filtration, concentration, extraction, distillation, sublimation, recrystallization and column chromatography. It is preferred that Soxhlet extraction with an organic solvent is incorporated into the purification step, as it is simple, in order to remove different impurities having different solubility from the compound, and thereby improve the purity of the compound.

### (Solubility of benzobis(thiadiazole) derivative)

The benzobis(thiadiazole) derivative of the present invention is generally soluble in various organic solvents, for example, alcohols such as methanol, ethanol, propanol, ethylene glycol, isobutanol, 2-butanol, 2-ethyl-1-butanol, n-octanol, benzyl alcohol, and terpineol; ketones such as acetone, methyl ethyl ketone, methyl isobutyl ketone, cyclopentanone, cyclohexanone, acetophenone, and isophorone: esters such as methyl acetate, ethyl acetate, butyl acetate, methyl benzoate, butyl benzoate, methyl salicylate ethyl malonate, 2-ethoxyethane acetate, and 2-methoxy-1-methylethyl acetate; amides such as N,N-dimethylformamide, N,N-dimethylacetamide, N,N-diethylformamide, N-methyl pyrrolidone, N-ethyl pyrrolidone, and hexamethylphosphoric triamide; ureas such as 1,3-dimethyl-2-imidazolidinone, and 1,3-dimethylimidazolidine-2,4-dione; sulfoxides such as dimethyl sulfoxide, and diethyl sulfoxide; sulfones such as sulfolane; nitriles such as acetonitrile, propionitrile, butyronitrile, and benzonitrile; lactones such as γ-butyrolactone; ethers such as diethyl ether, diisopropyl ether, tetrahydrofuran, 2-methyltetrahydrofuran, dioxane, tert-butyl methyl ether, anisole, phenetole, 1,2-dimethoxybenzene, 1,3-dimethoxybenzene, 1,4-dimethoxybenzene, 1,2-methylanisole, 1,3-methylanisole, 1,4-methylanisole, 1,2-methylene dioxybenzene, 2,3-dihydrobenzofuran, phthalan, octyloxybenzene, diphenyl ether, and ethylcellosolve; carbonates such as dimethyl carbonate, and 1,2-butylene glycol carbonate; thioethers such as thioanisole, and ethyl phenyl sulfide; aromatic hydrocarbons such as benzene, toluene, xylene, mesitylene, pseudocumene, hemimellitene, durene, isodurene, prehnitene, ethylbenzene, cumene, tert-butyl benzene, cyclohexyl benzene, triisopropyl benzene, phenyl acetylene, indane, methyl indane, indene, tetralin, naphthalene, 1-methyl naphthalene, 2-methyl naphthalene, phenyloctane, and diphenyl methane; phenols such as phenol, 1,2-cresol, 1,3-cresol, 1,4-cresol, 1,2-methoxyphenol, 1,3-methoxyphenol, and 1,4-methoxyphenol; halogenated aromatic hydrocarbons such as chlorobenzene, 1,2-dichlorobenzene, 1,3-dichlorobenzene, 1,2,4-trichlorobenzene, bromobenzene, 1,2-dibromobenzene, 1,3-dibromobenzene, 1,4-dichlorotoluene, 1-chloronaphthalene, 2,4-dichlorotoluene, 2-chloro-1,3-dimethylbenzene, 2-chlorotoluene, 2-chloro-1,4-dimethylbenzene, 4-chloro-1,2-dimethylbenzene, 2,5-dichlorotoluene, m-chlorotoluene, 1-chloro-2,3-dimethylbenzene, 4-(trifluoromethoxy) anisole, and trifluoromethoxybenzene; aliphatic hydrocarbons such as hexane, heptane, octane, cyclohexane, and limonene; halogenated aliphatic hydrocarbons such as dichloromethane, chloroform, carbon tetrachloride, 1,2-dichloroethane, 1,3-dichloropropane, and 1,2-dibromoethane; pyridines such as 2,6-dimethylpyridine, and 2,6-di-tert-butylpyridine.

The term "soluble in an organic solvent" used herein means that a compound has a solubility of preferably 0.03 wt% or more, more preferably 0.1 wt% or more for an organic solvent at normal pressure and a temperature of the boiling point or lower, preferably 80°C or lower, more preferably 15°C to 30°C or lower. It is not required that the benzobis(thiadiazole) derivative of the present invention be soluble in all organic solvents, and the derivative may be soluble in at least one of organic solvents as described above, for example. Also, the organic solvents include a mixed solvent of a plurality of organic solvents. The benzobis(thiadiazole) derivative of the present invention is not soluble in any one of organic solvent, but may be soluble in a mixed solvent.

Among the organic solvents as described above, halogenated aromatic hydrocarbons, aromatic hydrocarbons and halogenated aliphatic hydrocarbons may be suitably used as organic solvents from the viewpoint of the organic semiconductor film-forming properties and its semiconductor properties of the organic semiconductor ink of the present invention as described above.

Suitable halogenated aromatic hydrocarbons include chlorobenzene and 1,2-dichlorobenzene.

Suitable aromatic hydrocarbons include toluene, xylene, mesitylene, tetralin, cyclohexylbenzene, and 1-methylnaphthalene.

Suitable halogenated aliphatic hydrocarbons include chloroform and 1,2-dichloroethane.

Moreover, from the similar viewpoint as described above, suitable other organic solvents include methyl benzoate, methyl salicylate, anisole, 4-methylanisole, and meta-cresol.

### (Semiconductor properties of benzobis(thiadiazole) derivative)

The benzobis(thiadiazole) derivative of the present invention as described above has the benzobis(thiadiazole) ring represented by the general formula (1) or (2) as described above at center, which has cyclic imide structures annelated to an aromatic ring. Thus, the derivative has an excellent mobility of electron, and can be used as excellent n-type organic semiconductor materials.

In addition, the benzobis(thiadiazole) derivative dominantly exhibits the n-type semiconductor properties than p-type semiconductor properties, and therefore is very useful as organic semiconductor materials in a transistor device or the like.

The term "dominantly exhibits n-type semiconductor properties than p-type semiconductor properties" as used herein means when the conditions mentioned below are satisfied. The field-effect mobility of n-type is 10⁴ fold or more higher than the field-effect mobility of p-type.

### (Stability of benzobis(thiadiazole) derivative)

The benzobis(thiadiazole) derivative of the present invention has a very high stability to heat, and is not decomposed even when heated to 350°C. Also, the derivative has a high stability in the atmosphere, and it is possible to obtain a high mobility when making of a transistor device using the derivative in the atmosphere. Also, the produced transistor device has not change in the mobility of electron even after being left in the atmosphere.

### <Organic semiconductor ink>

As described above, the benzobis(thiadiazole) derivative of the present invention exhibits the n-type semiconductor properties, and is additionally stable in the atmosphere and generally soluble in an organic solvent, and therefore the benzobis(thiadiazole) derivative of the present invention can be dissolved in an organic solvent, and the resulting solution can be used as an organic semiconductor ink. These organic solvents may be used alone, or two or more solvents may be mixed and used.

When an organic semiconductor ink can be prepared, it is easy to handle and it can be stored. Additionally, although vapor deposition and coating are generally exemplified as a method of forming an organic semiconductor layer, the cost of vapor deposition is very high as compared with coating because a high-temperature heat source and a high vacuum are required for vapor deposition. When the organic semiconductor ink of the present invention is used, the cost of the organic semiconductor layer can be significantly reduced.

Additionally, the benzobis(thiadiazole) derivative of the present invention is stable in the atmosphere, and therefore the coating thereof may be performed in the atmosphere and there is no need to create an atmosphere of an inert gas such as argon gas. In view of this, cost reduction may be achieved using the organic semiconductor ink of the present invention.

The organic semiconductor ink of the present invention comprises one or more of the benzobis(thiadiazole) derivatives of the present invention, and may comprise one or more of other organic semiconductors. Further, the organic semiconductor ink of the present invention may comprise additives to control the properties of the ink such as an additive to adjust the viscosity of the ink, and an additive to control the hydrophilicity or the water-repellency of the ink, an anti-oxidizing agent, a light stabilizing agent, a surface conditioning agent (leveling agent), a surfactant, a storage stabilizing agent, a lubricating agent, a wettability improving agent, and a coupling agent.

The additives to adjust the viscosity of the ink include an insulating polymer compound. The insulating polymer compound as used herein is synthetic resin, plastic, synthetic rubber or the like, and specific examples thereof include polyethylene, polypropylene, polyvinyl chloride, polystyrene, polyester, phenol resin, acrylic resin, amide resin, nylon, vinylon, polyisoprene, polybutadiene, acrylic rubber, acrylonitrile rubber, and urethane rubber. By the addition thereof, the optimization of the viscosity of the organic semiconductor ink, and the improvement in the film-forming properties of the ink may be achieved.

The content of the benzobis(thiadiazole) derivative of the present invention in the organic semiconductor ink is not particularly limited, and may be appropriately selected. For example, the content may be from about 0.001 wt% to about 10 wt%, and may be preferably from about 0.01 wt% to about 1 wt% from the viewpoint of the film-forming properties. Additionally, in the cases where the organic semiconductor ink of the present invention comprises other organic semiconductors, the total content of the other organic semiconductors and the benzobis(thiadiazole) derivative of the present invention may be from 0.001 wt% to 10 wt%, for example. Also, the amount of the various additives as described above to be added may be appropriately selected from the conventionally known range.

Examples of the other organic semiconductor include, for example, polymer semiconductor compounds. The polymer semiconductor compound as used herein is a polymer compound characterized by exhibiting semiconductor properties, and specific examples thereof include polyacetylene polymer, polydiacetylene polymer, polyparaphenylene polymer, polyaniline polymer, polytriphenylamine polymer, polythiophene polymer, polypyrrole polymer, polyparaphenylenevinylene polymer, polyethylenedioxythiophene polymer, copolymers comprising naphthalenediimide as one component, copolymers comprising perylenediimide as one component, and copolymers comprising diketopyrrolopyrrole as one component.

Among these polymer semiconductor compounds, polyaniline polymer, polythiophene polymer, polypyrrole polymer, polyparaphenylenevinylene polymer, copolymers comprising naphthalenediimide as one component, copolymers comprising perylenediimide as one component, copolymers comprising diketopyrrolopyrrole as one component and the like are suitable.

Additional examples of the other organic semiconductor include, for example, low-molecular-weight semiconductor compounds other than the benzobis(thiadiazole) derivative of the present invention. The low-molecular-weight semiconductor compound as used herein is a low-molecular-weight compound characterized by exhibiting semiconductor properties, and specific examples thereof include acene, phenylenevinylene, triphenylamine, fluorene, azaacene, thienoacene, thiophene, benzothiophene, thienothiophene, thiazole, thiazolothiazole, tetrathiafulvalene, phthalocyanine, porphyrin, naphthalenediimide, perylenediimide, benzothiadiazole, naphthobisthiadiazole, diketopyrrolopyrrole, fullerene and these derivatives.

Among these low-molecular-weight semiconductor compounds, for example, acene, thienoacene, thiophene, thienothiophene, tetrathiafulvalene, naphthalenediimide, perylenediimide, diketopyrrolopyrrole, fullerene and these derivatives are suitable.

In addition, examples of the other organic semiconductor include, for example, organic semiconductors described in Chem. Rev., 2012, Vol. 112, pp. 2208-2267.

The organic semiconductor ink of the present invention may also comprise a conductive polymer compound as an additive component, as necessary. The conductive polymer compound as used herein is a polymer compound characterized by exhibiting electrical conductivity, and specific examples thereof include polyacetylene polymer, polydiacetylene polymer, polyparaphenylene polymer, polyaniline polymer, polytriphenylamine polymer, polythiophene polymer, polypyrrole polymer, polyparaphenylenevinylene polymer, polyethylenedioxythiophene polymer, and a mixture of polyethylenedioxythiophene and polystyrene sulfonic acid (generic name: PEDOT-PSS).

Among these conductive polymer compounds, polyacetylene polymer, polyparaphenylene polymer, polyaniline polymer, polytriphenylamine polymer, polythiophene polymer, polypyrrole polymer, and polyparaphenylenevinylene polymer and the like are suitable. The effects of the addition thereof include the improvement in the electric charge mobility, in addition to the optimization of the viscosity of the ink, the improvement in the film-forming properties of the ink and the like.

The organic semiconductor ink of the present invention may also comprise low-molecular-weight compounds as described below, as necessary, as an additive to control the properties of the ink. Specific examples thereof include aliphatic hydrocarbons such as decane, undecane, dodecane, tridecane, tetradecane, pentadecane, hexadecane, heptadecane, octadecane, nonadecane, and icosane; aliphatic alcohols such as hexanol, heptanol, octanol, nonanol, decanol, undecanol, dodecanol, tridecanol, tetradecanol, pentadecanol, hexadecanol, heptadecanol, octadecanol, nonadecanol, and eicosanol; aliphatic amines such as hexylamine, heptylamine, octylamine, nonylamine, decylamine, undecylamine, dodecylamine, tridecylamine, tetradecylamine, pentadecylamine, hexadecylamine, heptadecylamine, octadecylamine, nonadecylamine, and eicosylamine; aliphatic thiols such as hexanethiol, heptanethiol, octanethiol, nonanethiol, decanethiol, undecanethiol, dodecanethiol, tridecanethiol, tetradecanethiol, pentadecanethiol, hexadecanethiol, heptadecanethiol, octadecanethiol, nonadecanethiol, eicosanethiol, phenylmethanethiol, (2-methylphenyl)methanethiol, (3-methylphenyl)methanethiol, (4-methylphenyl)methanethiol, (2-fluorophenyl)methanethiol, (3-fluorophenyl)methanethiol, (4-fluorophenyl)methanethiol, and 2-phenylethanethiol; and aromatic thiols such as benzenethiol, 2-methylbenzenethiol, 3-methylbenzenethiol, 4-methylbenzenethiol, 2-ethylbenzenethiol, 3-ethylbenzenethiol, 4-ethylbenzenethiol, 2-aminobenzenethiol, 3-aminobenzenethiol, 4-aminobenzenethiol, 2-isopropylbenzenethiol, 3-isopropylbenzenethiol, 4-isopropylbenzenethiol, 2-(dimethylamino)benzenethiol, 3-(dimethylamino)benzenethiol, and 4-(dimethylamino)benzenethiol.

The effects of the addition thereof include the improvement in the electric charge mobility, in addition to the optimization of the viscosity of the organic semiconductor ink, the improvement in the film-forming properties of the ink and the like. Among these low-molecular-weight compounds, aliphatic thiols and aromatic thiols are suitable for the purpose of improving the wettability of the ink, and thereby improving the mobility.

A layer, or a thin film of the benzobis(thiadiazole) derivative of the present invention may be formed by coating of the organic semiconductor ink of the present invention as described above. The coating of the organic semiconductor ink of the present invention may be performed by any known methods, for example, spin-coating method, drop-casting method, casting method, Langmuir-Blodgett method, or the like. In addition, any known method commonly known as printing technique may be applied as the coating method, and the printing may be performed by, for example, ink-jet method, screen method, offset method, gravure method, flexographic method, microcontact method, or the like.

A layer, or a thin film comprising the benzobis(thiadiazole) derivative of the present invention (hereinafter, referred to as "organic semiconductor layer") is formed when the solvent component is removed from the organic semiconductor ink of the present invention after a substrate is coated on the ink. The conditions of the removal of the solvent component may be appropriately selected.

It is preferred that the solvent component be naturally-dried, or air-dried at room temperature, for example. Meanwhile, in the cases where the solvent has a high boiling point, and therefore is hard to remove, the solvent may be removed at around room temperature under a reduced pressure, or alternatively, the solvent may be removed by heating at about 50°C to about 200°C, or alternatively, the solvent may be removed by the combination of both of them and by heating under a reduced pressure.

In addition, for the purpose of improving the semiconductor properties of the layer or the thin film comprising the benzobis(thiadiazole) derivative of the present invention, the layer or thin film may be subjected to heat treatment. In this case, the conditions of the heat treatment may be appropriately selected, and examples thereof include a process in which the layer or the thin film is heated at a temperature of from about 50°C to about 250°C for 0.1 hour to 24 hours. The step may double as the solvent removal step.

In addition, for the purpose of improving the semiconductor properties of the layer or thin film, the layer or the thin film may be subjected to treatment by exposure to a vapor of a solvent.

Examples of the organic solvent used in this step include organic solvents as described above in which the benzobis(thiadiazole) derivative of the present invention may be dissolved, and preferred organic solvents are halogenated aromatic hydrocarbons, aromatic hydrocarbons and halogenated aliphatic hydrocarbons similarly to the case described above, and these suitable examples also are those similarly to the case described above. These organic solvents may be used alone, or two or more solvents may be mixed and used.

The solvent vapor exposure treatment step is performed, for example, by leaving the layer or the thin film comprising the benzobis(thiadiazole) derivative and a solvent, without the direct contact of the layer or thin film with the solvent (in form of liquid), in an enclosed space. In order to increase the amount of the solvent vapor, the solvent may be heated at a temperature of from about 40°C to about 150°C. Subsequent to the solvent vapor exposure treatment step, the drying step and heating treatment step similarly to those described above may be appropriately selected and performed in the step in order to remove the solvent attached to the layer or thin film as described above.

### <Organic electronic device>

The benzobis(thiadiazole) derivative of the present invention has an excellent mobility of electron (field-effect mobility), and therefore may be suitably used for organic electronic devices including an organic thin film transistor, an organic electroluminescence device, a display device, an organic thin film photovoltaic cell, a RFID tag, and a sensor, for example. All of these various devices comprise an organic layer comprising an organic semiconductor as a constituent member, and this organic layer comprises the benzobis(thiadiazole) derivative of the present invention.

Also, the benzobis(thiadiazole) derivative of the present invention may find extensive application in fields such as backlight, optical communication, electrophotography, illuminating light source, recording light source, exposing light source, reading light source, sign, signboard, and interior goods, as well as distribution management, stock management, commodity management, individual identification, and temperature measurement, pressure measurement, load measurement, brightness/darkness measurement, and biological information measurement. Hereinafter, the organic electronic devices are described individually.

### (Organic thin film transistor)

The organic thin film transistor (hereinafter, referred to as "organic TFT") of the present invention will be described below. The organic thin film transistor of the present invention is the one in which an organic semiconductor layer comprises the benzobis(thiadiazole) derivative as described above. It is effective to use the benzobis(thiadiazole) derivative of the present invention for a semiconductor layer of an organic TFT, because the orientation direction of the molecule may be readily aligned and the high field-effect mobility may be achieved.

Any known structure and any known material may be used for the organic thin film transistor of the present invention, except that the organic semiconductor layer comprises the benzobis(thiadiazole) derivative of the present invention.

It is preferred that the thickness of the organic semiconductor layer be thin, as long as the layer does not lose its necessary function. The thickness of the organic semiconductor layer to perform its necessary function is generally 1 nm to 10µm, preferably 5 nm to 5 µm, and more preferably 10 nm to 1 µm.

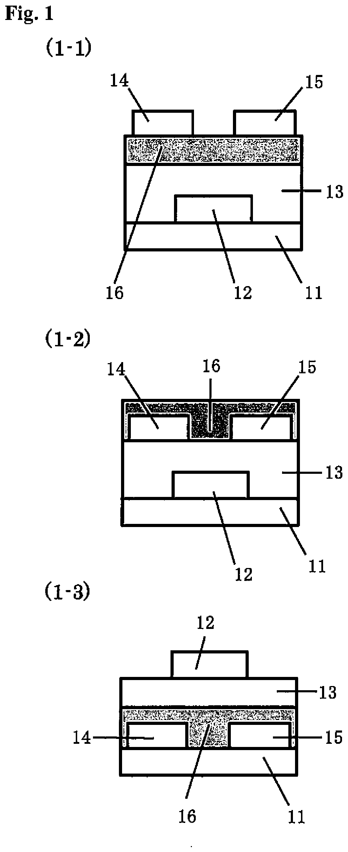

Fig. 1 shows one example of the layer configuration of the organic TFT of the present invention. The organic TFT shown in Fig. 1 (1-1) has a bottom gate-top contact structure, and is formed by laminating a gate electrode 12, a gate insulating layer 13, an organic semiconductor layer 16, and a drain electrode 14 and a source electrode 15, in this order, on a substrate 11.

As a structure other than the bottom gate-top contact structure, the organic TFT shown in Fig. 1 (1-2) and the organic TFT shown in Fig. 1 (1-3) have a bottom gate-bottom contact structure and a top gate-bottom contact structure, respectively. From the viewpoint of miniaturization and integration of the transistor device, a bottom gate-bottom contact structure or a top gate- bottom contact structure is generally suitable. Each symbol in Figs. 1 (1-2) and (1-3) represents the same as in Fig. 1 (1-1).

When the organic semiconductor molecule constituting the organic semiconductor layer 16 has a higher uniformity of molecular arrangement, better transistor characteristics are shown and therefore it is preferred that an interface contacting with the organic semiconductor thin film may be controlled for the purpose of controlling the uniformity of molecular arrangement of the organic semiconductor molecule constituting the organic semiconductor layer 16. The interface as used herein is specifically the one between the gate insulating layer 13 and the organic semiconductor layer 16, and the one between the drain electrode 14 and source electrode 15 and the organic semiconductor layer 16.

As the substrate 11 in the organic TFT, substrate materials shown in the following Group A may be used.

Group A is a sheet or film made from silicon based inorganic compounds, and a sheet or film made from polymer compounds.

Specifically, the substrate materials shown in Group A as used herein represent the following materials. The silicon based inorganic compound is glass, quartz, silicon and ceramic, and the polymer compound is polyimide, polyethylene terephthalate, polyethylene naphthalate, polyethylene, polypropylene, polyvinyl chloride, polyparaxylylene, polymethyl methacrylate, polyester, polyamide, polycarbonate, polyacrylate, polyether sulfone, polyurethane, polycarbon fluoride resins such as polytetrafluoroethylene, silicone resins such as polysiloxane, melamine resins, phenol resins, epoxy resins, urea resins, and rubbers. Also, materials in which two or more of these materials chemically or physically are mixed are encompassed in the substrate materials.

As mentioned above, benzobis(thiadiazole) derivative used in the present invention is generally soluble in an organic solvent, and an organic semiconductor layer may be formed by dissolving the benzobis(thiadiazole) derivative in a solvent to provide an organic semiconductor ink, and applying the ink. An organic semiconductor layer may be formed at a relatively low temperature of 200°C or lower by a coating method. Thus, an organic thin film transistor may be produced at a relatively low temperature process if other elements such as an electrode can be formed in an environment wherein the temperature is not high. In this case, a material having a low heat resistance may be used as the substrate 11.

Thus, the present invention has allowed the use of various plastic materials having a relatively low heat resistance as the substrate 11, and therefore has allowed the use of a material having flexibility. In this case, the substrate 11 may be a flexible substrate, thereby allowing the arrangement of the organic TFT on a curved surface, or the like, as well as on a plane surface, and enhancing the degree of freedom of the design of an organic electronic device comprising an organic TFT in its entirety. For this purpose, polyimide, polyethylene terephthalate, polyethylene naphthalate, polyethylene, polypropylene, polyvinyl chloride, polyparaxylylene, polymethyl methacrylate, polyester, polyamide, polycarbonate, polyacrylate, polyether sulfone, polyurethane, polycarbon fluoride resins such as polytetrafluoroethylene, silicone resins such as polysiloxane, melamine resins, phenol resins, epoxy resins, and urea resins are suitable among Group A.

From the viewpoint of the flexibity and transistor production in the low temperature process, in particular, polyethylene terephthalate, polyethylene naphthalate, polyethylene, polypropylene, polyvinyl chloride are suitable among Group A. On the other hand, from the viewpoint of the flexibity and the dimension stability of the substrate in the transistor production process, polyimide is suitable among Group A.

As a gate electrode 12, drain electrode 14 and source electrode 15, materials shown in the following Group B may be used. In the gate electrode, drain electrode and source electrode, electrode materials selected from Group B may be same or different.

Group B encompasses metals, metallic oxides, carbon materials, conductive polymer compounds, and silicon materials.

Specifically, electrode materials in Group B as used herein represent the following materials. Metals are gold, silver, copper, platinum, aluminium, tungsten, chromium, molybdenum, iron, zinc, tantalum, magnesium, calcium, lithium, cobalt, indium, tin, silicon, and nickel; metallic oxides are tin-doped indium oxide, indium oxide, tin oxide, and zinc oxide; carbon materials are graphite, and carbon nanotube; conductive polymer compounds are polyaniline, polythiophene, polypyrrole, and PEDOT/PSS (a mixture of polyethylenedioxythiophene and polystyrene sulfonic acid); and silicon materials are polysilicon, and amorphous silicon. Also, materials in which two or more of these materials chemically or physically are mixed are encompassed in Group B.

Among Group B, gold, silver, copper, aluminium as metals, carbon nanotube as carbon materials, and PEDOT/PSS as conductive polymer compounds are suitable.

The gate electrode or the like may be formed by well-known film-formation methods such as vacuum deposition, electron-beam evaporation deposition, RF sputtering and printing using the materials shown in Group B.

By modifying the surface of the drain electrode 14 and source electrode 15, an interface of the drain electrode 14 and source electrode 15 with the organic semiconductor layer 16 may be controlled. This modification is suitable for the organic TFT shown in Fig. 1 (1-2) having a bottom gate-bottom contact structure and the organic TFT shown in Fig. 1 (1-3) having a top gate-bottom contact structure.

Thus, at least one electrode material selected from the Group B as described herein is preferably used with at least one electrode modifying material selected from the following Group C.

Group C encompasses organic compounds, alkali metal salts, and metallic oxides.

Specifically, electrode modifying materials in Group C represent the following materials. Organic compounds are aliphatic thiols, aromatic thiols, aliphatic alcohols, aromatic phenols, aliphatic amines, aromatic amines, aliphatic carboxylic acids, aromatic carboxylic acids, aliphatic phosphonic acids, aliphatic phosphoric acids, disulfides, chlorosilanes, alkoxysilanes, and tetracyanoquinodimethane fluoride; alkali metal salts are lithium fluoride, lithium carbonate, cesium fluoride, and cesium carbonate; metallic oxides are molybdenum oxide, and tungsten oxide. Also, materials in which two or more of these materials chemically or physically are mixed are encompassed in Group C.

The surface of the drain electrode 14 and source electrode 15 may be modified by the following methods or the like using the materials selected from Group C:
- subjecting the drain electrode 14 and source electrode 15 to treatment by exposure to a vapor of materials selected from Group C.
- preparing a solution of materials selected from Group C, coating the solution onto the drain electrode 14 and source electrode 15, and removing the solvent.
- preparing a solution of materials selected from Group C, impregnating the drain electrode 14 and source electrode 15 into the solution, adsorbing the materials shown in Group C onto the surface of the drain electrode 14 and source electrode 15, and removing the solvent.

Among the materials described in Group C, suitable examples of the aliphatic thiols include, for example, hexanethiol, heptanethiol, octanethiol, nonanethiol, decanethiol, undecanethiol, dodecanethiol, tridecanethiol, tetradecanethiol, pentadecanethiol, hexadecanethiol, heptadecanethiol, octadecanethiol, nonadecanethiol, icosanethiol, phenylmethanethiol, (2-methylphenyl)methanethiol, (3-methylphenyl)methanethiol, (4-methylphenyl)methanethiol, (2-fluorophenyl)methanethiol, (3-fluorophenyl)methanethiol, (4-fluorophenyl)methanethiol, 2-phenylethanethiol, 3,3,4,4,5,5,6,6,6-nonafluoro-1-hexanethiol, 3,3,4,4,5,5,6,6,7,7,8,8,8-tridecafluoro-1-octanethiol, and 3,3,4,4,5,5,6,6,7,7,8,8,9,9,10,10,10-heptadecafluoro-1-decanethiol.

Suitable examples of the aromatic thiols include, for example, benzenethiol, 2-methylbenzenethiol, 3-methylbenzenethiol, 4-methylbenzenethiol, 2-ethylbenzenethiol, 3-ethylbenzenethiol, 4-ethylbenzenethiol, 2-aminobenzenethiol, 3-aminobenzenethiol, 4-aminobenzenethiol, 2-isopropylbenzenethiol, 3-isopropylbenzenethiol, 4-isopropylbenzenethiol, 2-(dimethylamino)benzenethiol, 3-(dimethylamino)benzenethiol, 4-(dimethylamino)benzenethiol, 2-methyl-3-furanthiol, 5-methyl-2-furanthiol, 2-thiophenethiol, and 3-thiophenethiol.

Suitable examples of the aliphatic alcohols include, for example, hexanol, heptanol, octanol, nonanol, decanol, undecanol, dodecanol, tridecanol, tetradecanol, pentadecanol, hexadecanol, heptadecanol, octadecanol, nonadecanol, and icosanol.

Suitable examples of the aromatic phenols include, for example, phenol, 2-methylphenol, 3-methylphenol, 4-methylphenol, 2-ethylphenol, 3-ethylphenol, 4-ethylphenol, 2-aminophenol, 3-aminophenol, 4-aminophenol, 2-isopropylphenol, 3-isopropylphenol, 4-isopropylphenol, 2-dimethylaminophenol, 3-dimethylaminophenol, 4-dimethylaminophenol, 4-propylphenol, 4-butylphenol, 4-pentylphenol, 4-hexylphenol, 4-heptylphenol, 4-octylphenol, 4-nonylphenol, 4-decylphenol, 4-undecylphenol, 4-dodecylphenol, 4-tridecylphenol, 4-tetradecylphenol, 4-pentadecylphenol, 4-hexadecylphenol, 4-heptadecylphenol, 4-octadecylphenol, 4-nonadecylphenol, and 4-icosylphenol.

Suitable examples of the aliphatic amines include, for example, hexylamine, heptylamine, octylamine, nonylamine, decylamine, undecylamine, dodecylamine, tridecylamine, tetradecylamine, pentadecylamine, hexadecylamine, heptadecylamine, octadecylamine, nonadecylamine, and icosylamine.

Suitable examples of the aromatic amines include, for example, aniline, 2-methylaniline, 3-methylaniline, 4-methylaniline, 2-ethylaniline, 3-ethylaniline, 4-ethylaniline, 1,2-phenylenediamine, 1,3-phenylenediamine, 1,4-phenylenediamine, 2-isopropylaniline, 3-isopropylaniline, 4-isopropylaniline, 4-propylbenzeneamine, 4-butylbenzeneamine, 4-pentylbenzeneamine, 4-hexylbenzeneamine, 4-heptylbenzeneamine, 4-octylbenzeneamine, 4-nonylbenzeneamine, 4-decylbenzeneamine, 4-undecylbenzeneamine, 4-dodecylbenzeneamine, 4-tridecylbenzeneamine, 4-tetradecylbenzeneamine, 4-pentyldecylbenzeneamine, 4-hexadecylbenzeneamine, 4-heptadecylbenzeneamine, 4-octadecylbenzeneamine, 4-nonadecylbenzeneamine, and 4-icosylbenzeneamine.

Suitable examples of aliphatic carboxylic acids include, for example, hexanoic acid, heptanoic acid, octanoic acid, nonanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, and icosanoic acid.

Suitable examples of the aromatic carboxylic acids include, for example, benzoic acid, 4-methyl benzoate, 4-ethyl benzoate, 4-propyl benzoate, 4-butyl benzoate, 4-pentyl benzoate, 4-hexyl benzoate, 4-heptyl benzoate, 4-octyl benzoate, 4-nonyl benzoate, 4-decyl benzoate, 4-undecyl benzoate, 4-dodecyl benzoate, 4-tridecyl benzoate, 4-tetradecyl benzoate, 4-pentadecyl benzoate, 4-hexadecyl benzoate, 4-heptadecyl benzoate, 4-octadecyl benzoate, 4-nonadecyl benzoate, and 4-icosyl benzoate.

Suitable examples of the aliphatic phosphonic acid include, for example, hexylphosphonic acid, heptylphosphonic acid, octylphosphonic acid, nonylphosphonic acid, decylphosphonic acid, undecylphosphonic acid, dodecylphosphonic acid, tridecylphosphonic acid, tetradecylphosphonic acid, pentadecylphosphonic acid, hexadecylphosphonic acid, heptadecylphosphonic acid, octadecylphosphonic acid, nonadecylphosphonic acid, and icosylphosphonic acid.

Suitable examples of the aliphatic phosphoric acid include, for example, hexylphosphoric acid, heptylphosphoric acid, octylphosphoric acid, nonylphosphoric acid, decylphosphoric acid, undecylphosphoric acid, dodecylphosphoric acid, tridecylphosphoric acid, tetradecylphosphoric acid, pentadecylphosphoric acid, hexadecylphosphoric acid, heptadecylphosphoric acid, octadecylphosphoric acid, nonadecylphosphoric acid, and icosylphosphoric acid.

Suitable examples of the disulfides include, for example, dihexyldisulfide, diheptyldisulfide, dioctyldisulfide, dinonyldisulfide, didecyldisulfide, diundecyldisulfide, didodecyldisulfide, ditridecyldisulfide, ditetradecyldisulfide, dipentadecyldisulfide, dihexadecyldisulfide, diheptadecyldisulfide, dioctadecyldisulfide, dinonadecyldisulfide, diicosyldisulfide, and 2,2'-dithienyldisulfide.

Suitable examples of the chlorosilanes include, for example, hexyltrichlorosilane, heptyltrichlorosilane, octyltrichlorosilane, nonyltrichlorosilane, decyltrichlorosilane, undecyltrichlorosilane, dodecyltrichlorosilane, tridecyltrichlorosilane, tetradecyltrichlorosilane, pentadecyltrichlorosilane, hexadecyltrichlorosilane, heptadecyltrichlorosilane, octadecyltrichlorosilane, nonadecyltrichlorosilane, icosyltrichlorosilane, phenyltrichlorosilane, 4-methylphenyltrichlorosilane, 4-ethylphenyltrichlorosilane, 4-propylphenyltrichlorosilane, 4-isopropylphenyltrichlorosilane, phenylmethyltrichlorosilane, phenylethyltrichlorosilane, phenylpropyltrichlorosilane, phenylbutyltrichlorosilane, phenylpentyltrichlorosilane, phenylhexyltrichlorosilane, phenylheptyltrichlorosilane, phenyloctyltrichlorosilane, pentafluorophenyldimethylchlorosilane, 3-(pentafluorophenyl)propyldimethylchlorosilane, and 3-(pentafluorophenyl)propyltrichlorosilane.

Suitable examples of the alkoxysilanes include, for example, hexyltrimethoxysilane, heptyltrimethoxysilane, octyltrimethoxysilane, nonyltrimethoxysilane, decyltrimethoxysilane, undecylmethoxysilane, dodecyltrimethoxysilane, tridecyltrimethoxysilane, tetradecyltrimethoxysilane, pentadecyltrimethoxysilane, hexadecyltrimethoxysilane, heptadecyltrimethoxysilane, octadecyltrimethoxysilane, nonadecyltrimethoxysilane, icosyltrimethoxysilane, phenyltrimethoxysilane, 4-methylphenyltrimethoxysilane, 4-ethylphenyltrimethoxysilane, 4-propylphenyltrimethoxysilane, 4-isopropylphenyltrimethoxysilane, phenylmethyltrimethoxysilane, phenylethyltrimethoxysilane, phenylpropyltrimethoxysilane, phenylbutyltrimethoxysilane, phenylpentyltrimethoxysilane, phenylhexyltrimethoxysilane, phenylheptyltrimethoxysilane, phenyloctyltrimethoxysilane, pentafluorophenyldimethylethoxysilane, and 11-(pentafluorophenoxy)undecanyltrimethoxysilane.

By modifying the surface of the drain electrode 14 and source electrode 15, an interface of the drain electrode 14 and source electrode 15 with the organic semiconductor layer 16 may be controlled. This modification is suitable for the organic TFT shown in Fig. 1 (1-2) having a bottom gate-bottom contact structure and the organic TFT shown in Fig. 1 (1-3) having a top gate-bottom contact structure. In addition to the methods using the materials selected from Group C for this modification, treatments which can change the physical state or chemical composition of the electrode surface such as chemical etching treatment, physical etching treatment, plasma treatment, and UV ozone treatment may be used.

Materials shown in the following Group D may be used as a gate insulating layer 13. Also, the gate insulating layer 13 may be a laminated structure comprising a plurality of layers consisting of two or more materials selected from the following Group D.

Group D encompasses metallic oxides, metallic nitrides, and non-conductive polymer compounds.

Specifically, materials in Group D as used herein represent the following materials. Metallic oxides and metallic nitrides are SiO₂, Si₃N₄, SiON, Al₂O₃, and Ta₂O₅, non-conductive polymer compounds are polyimide, polyethylene terephthalate, polyethylene naphthalate, polyvinylphenol, polyethylene, polystyrene, polymethyl methacrylate resin, divinyltetramethylsiloxane benzocyclobutene resin, polypropylene, polyvinyl chloride, polyparaxylylene, polyester, polyamide, polycarbonate, polyacrylate, polyether sulfone, polyurethane, polycarbon fluoride resins such as polytetrafluoroethylene, silicone resins such as polysiloxane, nylon resins, vinylon resins, melamine resins, phenol resins, epoxy resins, urea resins; plastic materials such as cyclobutene containing polymer, cycloolefin containing polymer, fluorene containing polymer, and silsesquioxane containing polymer; and rubbers such as natural rubbers, polyisoprene rubbers, polybutadiene, acrylic rubbers, acrylonitrile rubbers, urethane rubbers. Also, materials in which two or more of these materials chemically or physically are mixed are encompassed in Group D.

Among Group D, SiO₂ and Al₂O₃ as metallic oxides, and polyimide, polycarbon fluoride resin, divinyltetramethylsiloxane benzocyclobutene resin, polyparaxylylene and a complex resin of polyvinylphenol and melamine resin as non-conductive polymer compounds are suitable.

Also, when two or more of the materials selected from Group D are laminated to make a gate insulating layer, a combination of lamination of polystyrene on SiO₂, polymethyl methacrylate on SiO₂, polycarbon fluoride resin on SiO₂, a complex resin of polyvinylphenol and melamine resin on SiO₂, and divinyltetramethylsiloxane benzocyclobutene resin on SiO₂ are suitable examples thereof.

These may be formed by well-known film-formation methods as listed for the gate electrode 12.

By modifying the surface of the gate insulating layer 13, an interface of the organic semiconductor layer 16 with the gate insulating layer 13 may be controlled. This modification is suitable for the organic TFT shown in Fig. 1 (1-1) having a bottom gate-top contact structure and the organic TFT shown in Fig. 1 (1-2) having a bottom gate-bottom contact structure.

Thus, at least one gate insulating layer material selected from the Group D as described above is preferably used with at least one gate insulating layer-modifying material selected from the following Group E.

Group E encompasses organic compounds. Specifically, materials in Group E as used herein represent the following materials. Aliphatic thiols, aromatic thiols, aliphatic alcohols, aromatic phenols, aliphatic amines, aromatic amines, aliphatic carboxylic acids, aromatic carboxylic acids, aliphatic phosphonic acids, aliphatic phosphoric acids, disilazanes, disulfides, chlorosilanes, and andalkoxysilanes. Also, materials in which two or more of these materials chemically or physically are mixed are encompassed in group E.

Among these, aliphatic thiols, aromatic thiols, aliphatic alcohols, aromatic phenols, aliphatic amines, aromatic amines, aliphatic carboxylic acids, aromatic carboxylic acids, aliphatic phosphonic acids, aliphatic phosphoric acids, disulfides, chlorosilanes, and alkoxysilanes are similarly to those in group C described above.

Suitable examples of the disilazanes include hexamethyldisilazane.

The surface of the gate insulating layer 13 may be modified by the following methods or the like using the materials selected from group E:
- subjecting the gate insulating layer 13 to treatment by exposure to a vapor of materials selected from Group E.
- preparing a solution of materials selected from Group E, coating the solution onto the gate insulating layer 13, and removing the solvent.
- preparing a solution of materials selected from Group E, impregnating the gate insulating layer 13 into the solution, adsorbing the materials selected from Group E onto the surface of the gate insulating layer 13, and removing the solvent.

As mentioned above, the electrode modifying materials in the Group C may be used with at least one electrode material selected from the Group B, and the gate insulating layer-modifying materials in the Group E may be used with at least one gate insulating layer material selected from the Group D. Thus, the organic thin film transistor preferably comprises at least one electrode modifying material selected from Group C, and at least one gate insulating layer-modifying material from the Group E. However, the organic TFT may be comprise any one of the electrode modifying material in the Group C and the gate insulating layer-modifying material in the Group E accordingly the purpose and application of thereof.

By modifying the surface of the gate insulating layer 13, an interface of the organic semiconductor layer 16 with the gate insulating layer 13 may be controlled. This modification is suitable for the organic TFT shown in Fig. 1 (1-1) having a bottom gate-top contact structure and the organic TFT shown in Fig. 1 (1-2) having a bottom gate-bottom contact structure. In addition to the methods using the materials selected from Group E for this modification, treatments which can change the physical state or chemical composition of the surface of the gate insulating layer such as chemical etching treatment, physical etching treatment, plasma treatment, and UV ozone treatment may be used.

In the organic thin film transistor of the present invention, the organic semiconductor layer 16 comprise one or more of the benzobis(thiadiazole) derivative used in the present invention1, and may be formed by well-known film-formation methods such as vacuum deposition and spin-coating method. The benzobis(thiadiazole) derivative used in the present invention is generally soluble in an organic solvent, and therefore the organic semiconductor layer 16 can be formed by a coating method (or printing). Also, the organic semiconductor layer 16 may comprise one or more of other organic compounds.

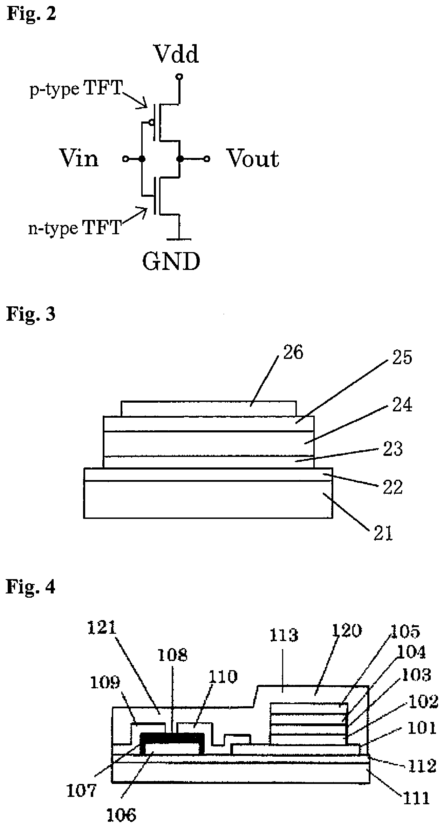

The organic TFT of the present invention generally has good n-type properties, and therefore may be combined with a p-type organic TFT to form the complementary inverter (logic inverting) circuit (The circuit is also referred to as "NOT circuit" sometimes) shown in Fig. 2. In the figure, Vin represents an input signal line, Vout represents an output signal line, Vdd represents a power supply line, and GND represents an earth connection. Additionally, in the figure, the upper transistor is a p-type TFT, and the lower transistor is an n-type TFT.

In order to activate the circuit, an electric potential difference capable of driving the transistors is applied to Vdd relative to GND. When Vin is at the same electrical potential as GND, the p-type TFT is in ON-state and the n-type TFT is in OFF-state, and therefore almost the same electrical potential as Vdd is output to Vout. On the other hand, when Vin is at the same electrical potential as Vdd, the p-type TFT is in OFF-state and the n-type TFT is in ON-state, and the electrical potential of Vout is almost the same as the electrical potential of GND. Thus the electrical potential opposite to Vin is output to Vout, and therefore the circuit is referred to as "inverting circuit". The circuit is a basic circuit which constitutes a digital logic circuit. The circuit is preferably used in view of the construction of a logic circuit with a low electric power consumption, because only a small electric current flows only at the moment of logic inversion.

The organic TFT of the present invention, which has good n-type properties, may also be combined with a p-type organic TFT to form a NAND circuit, a NOR circuit, a flip-flop circuit, or the like. These circuits may be combined and integrated based on a design according to the purpose to form an integrated logic circuit, which may be applied to a display circuit, a RFID circuit, a sensor circuit, and the like.

### (Organic electroluminescence device)

The organic electroluminescence device (hereinafter, referred to as "organic EL device") of the present invention will be described below. The organic EL device of the present invention is the one in which a hole transport layer and/or an electron transport layer as a constituent member comprise the benzobis(thiadiazole) derivative of the present invention. It is effective to use the benzobis(thiadiazole) derivative of the present invention for, in particular, an electron transport layer of an organic EL device, because the benzobis(thiadiazole) derivative has excellent electron transport properties.

Any known structure and any known material may be used for the organic EL device of the present invention, except that the hole transport layer and/or the electron transport layer comprises the benzobis(thiadiazole) derivative of the present invention.

The organic EL device is a device in which organic compound layers, including a luminescent layer as well as a hole transport layer and/or an electron transport layer, are formed between an anode and a cathode on a substrate. The organic EL device is typically configured to have a device structure of (substrate /anode /hole transport layer /luminescent layer /cathode), (substrate /anode /luminescent layer /electron transport layer /cathode), (substrate /anode /hole transport layer /luminescent layer /electron transport layer /cathode), or the like.

Fig.3 shows one example of the layer configuration of the organic EL device of the present invention. The organic EL device shown in Fig. 3 is formed by laminating an anode 22, a hole transport layer 23, a luminescent layer 24, an electron transport layer 25, and a cathode 26, in this order, on a substrate 21.

When a predetermined direct voltage is applied between the anode 22 and the cathode 26 of the organic EL device configured as described above, light with high intensity is emitted from the luminescent layer 24. The mechanism of the light emission is considered as follows.

Specifically, when a predetermined direct voltage is applied between the two layers as described above, holes which flow from the anode 22 to the hole transport layer 23 are transported to the luminescent layer 24. Meanwhile, electrons which are injected from the cathode 26 to the electron transport layer 25 are transported to the luminescent layer 24. In the luminescent layer 24, electrons diffuse and migrate, and recombine with holes to achieve a state of electrically neutralization. When the recombination occurs, a certain energy is released, and the organic luminescent material in the luminescent layer 24 is excited to the excitation state by the energy. When the material returns to the ground state from the excited state, light is emitted.

In particular, when the benzobis(thiadiazole) derivative of the present invention, which has the high field-effect mobility, is used for the electron transport layer 25 of the organic EL device, electrons may be efficiently injected into the luminescent layer, and therefore the luminous efficiency may be enhanced.

As the substrate 21 as described above, transparent materials such as glass and plastics may be used, for example. The hole transport layer 23 and electron transport layer 25 may be formed by a coating method using the organic semiconductor ink of the present invention, and a flexible substrate may be used as the substrate 21 in similarly to the case of the organic TFT described above.

As the anode 22 as described above, a light-transmission material is generally used. Specifically, tin-doped indium oxide (ITO), indium oxide, tin oxide, indium oxide, and zinc oxide alloy are preferably used. A thin film of metal such as gold, platinum, silver, and magnesium alloy may also be used. In addition, organic materials such as polyaniline, polythiophene, polypyrrole, and derivatives thereof may also be used. The anode 22 may be formed by well-known film-formation methods such as vacuum deposition, electron-beam evaporation deposition, RF sputtering, and coating (printing).

As the cathode 26 as described above, alkali metals such as Li, K and Na, and alkali-earth metals such as Mg and Ca, which have small work function, are preferably used, from the viewpoint of the electron injection properties. In addition, Al which is stable, and the like are also preferably used. In order to achieve both stability and electron injection properties, the cathode may be a layer comprising two or more materials, and the materials are described in detail, for example, in JP-A-H02-15595, JP-A-H05-121172, etc. The cathode 26 may be formed by well-known film-formation methods such as vacuum deposition, electron-beam evaporation deposition, and RF sputtering.

As the luminescent layer 24 as described above, a host material such as quinolinol complex and aromatic amine which is doped with (doping) a coloring material such as coumarin derivatives, DCM, quinacridone and rubrene may be preferably used. The luminescent layer 24 may also be formed from a host material only. In addition, a high-efficiency organic EL device may be produced by forming the luminescent layer 24 doped with iridium metal complex. The luminescent layer 24 may be formed by well-known film-formation methods such as vacuum deposition, sputtering, and coating (printing).

The hole transport layer 23 and/or the electron transport layer 25 comprise the benzobis(thiadiazole) derivative of the present invention. In the cases where the benzobis(thiadiazole) derivative of the present invention is used, the hole transport layer 23 and the electron transport layer 25 may be suitably formed by the coating method using the organic semiconductor ink of the present invention.

In the cases where the benzobis(thiadiazole) derivative of the present invention is not used for the hole transport layer 23, materials such as N,N'-bis(3-methylphenyl)-N,N'-bis(phenyl)-benzidine (TPD), N,N'-bis(naphthalen-1-yl)-N,N'-bis(phenyl)-2,2'-dimethyl benzidine (α-NPD), and 2,2-bis(3-(N,N-di-p-tolylamino)phenyl)biphenyl (3DTAPBP), for example, may be used as the hole transport layer 23.

In the cases where the benzobis(thiadiazole) derivative of the present invention is not used for the electron transport layer 25, materials such as 2-(4-biphenyl)-5-(4-tert-butylphenyl)-1,3,4-oxazole (PBD), 1,3-bis[2-(4-tert-butylphenyl)-1,3,4-oxadiazo-5-yl]benzene (OXD-7), and 2,2',2"-(1,3,5-benzinetriyl)-tris(1-phenyl-1-H-benzimidazole) (TPBi), for example, may be used as the electron transport layer 25.

As the method of film-formation of the hole transport layer 23 and the electron transport layer 25, the methods as listed for the method of film-formation of the luminescent layer 24 may be used. The thicknesses of the hole transport layer 23 and the electron transport layer 25 may be appropriately selected from the conventionally known range, and may be generally 1 nm to 1 µm, preferably 1 nm to 100 nm.

The organic EL device of the present invention may be configured to comprise an electron injection layer, a hole injection layer, an electron blocking layer, a hole blocking layer, a protective layer, and the like, in addition to the layers as described above. These layers may be formed by the methods as listed for the method of film-formation of the luminescent layer 24.

### (Display device)

The display device of the present invention will be described below. The display device of the present invention is the one which comprises an organic EL device and an organic TFT electrically connected to the organic EL device and in which the driving (switching transistor) and lighting (driving transistor) of the organic EL device is controlled by the organic TFT, and the organic TFT is the organic TFT of the present invention as described above, or the organic EL device is the organic EL device of the present invention as described above. As for the display device of the present invention, it is preferred that the organic TFT is the organic TFT of the present invention, and the organic EL device is the organic EL device of the present invention from the viewpoint of the high field-effect mobility.

Fig.4 shows one configuration example of the display device of the present invention. The display device shown in Fig. 4 comprises an organic EL device 120 comprising a cathode 101, an electron transport layer 102, a luminescent layer 103, a hole transport layer 104 and an anode 105, and an organic TFT 121 comprising a gate electrode 106, a gate insulating layer 107, an organic semiconductor layer 108, a source electrode 109 and a drain electrode 110 on a substrate 111 with a barrier layer 112 therebetween. Additionally, the upper part of the layer structure is coated with a protective film 113.

The display device has a structure in which the cathode 101 of the organic EL device 120 (electrode closer to the substrate 111) is electrically connected to the drain electrode 110 of the organic TFT 121. When a voltage is applied to the gate electrode 106, an electric current flows between the source electrode and the drain electrode, and the organic EL device 120 emits light. In addition, the display device may have a structure in which the anode is electrically connected to the drain electrode of the organic TFT.

In the present invention, it is preferred that the organic TFT, and the organic EL device which is driven/lighted by the organic TFT are the organic TFT of the present invention, and the organic EL device of the present invention, both of which comprise the benzobis(thiadiazole) derivative of the present invention, as described above. However, one of them may comprise no benzobis(thiadiazole) derivative of the present invention, and may be formed from a known material and have a known structure.

In addition, an active-matrix display device may be formed by arranging devices (pixels) as shown in Fig. 4, in which the organic TFT for controlling for the driving/lighting and the organic EL device are combined, in a matrix form. The active-matrix display device has the advantages of having a low possibility of the application of unnecessary voltage to a non-selected point even in the case of a great number of pixels; having a low possibility of the field efficiency reduction and deterioration even in high-duty operation; and having excellent response properties.

Any known structure and any known material may be used for the display device (display) of the present invention, except that the organic TFT of the present invention and/or the organic EL device of the present invention are employed. The display device may be produced by any known method.

### (Organic thin film photovoltaic cell)

The organic thin film photovoltaic cell (hereinafter, referred to as "organic PV device") of the present invention will be described below. The organic PV device of the present invention is the one having an anode, a charge separation layer comprising a hole transport material and an electron transport material, and a cathode on a substrate, the charge separation layer comprising the benzobis(thiadiazole) derivative of the present invention.

Moreover, the organic PV device of the present invention includes the one having an anode, the charge separation layer, a hole transport layer and/or an electron transport layer, an anode on a substrate, the hole transport layer and/or electron transport layer comprising the benzobis(thiadiazole) derivative of the present invention.

It is effective to use the benzobis(thiadiazole) derivative of the present invention for, in particular, an charge separation layer or an electron transport layer of an organic PV device, because the benzobis(thiadiazole) derivative has electron transport properties.

In addition to the configuration as described above, any known structures and materials may be used in the organic PV device of the present invention.

The organic PV device is a device in which at least one organic compound layer, including a charge separation layer, are formed between an anode and a cathode on a substrate. The organic PV device is typically configured to have a device structure of (substrate /anode /charge separation layer /cathode), (substrate /anode /charge separation layer /electron transport layer /cathode), (substrate /anode /hole transport layer /charge separation layer /electron transport layer /cathode) or the like.

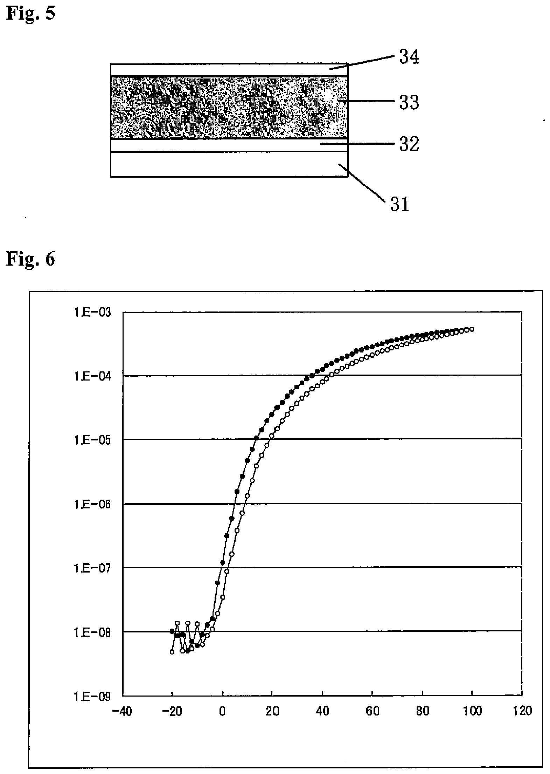

Fig.5 shows one example of the layer configuration of the organic PV device of the present invention. The organic PV device shown in Fig. 5 is formed by laminating an anode 32, a charge separation layer 33, and a cathode 34, in this order, on a substrate 31.

When the organic PV device configured as described above is irradiated with light, holes and electrons are generated in the charge separation layer 33, and an electric current is taken out if the anode 32 is connected to the cathode 34. The mechanism of the generation of electricity is considered as follows.

Specifically, when the charge separation layer 33 as described above is irradiated with light, the light is absorbed, and the organic molecule is excited by the energy to provide charge separation, and generate holes and electrons. The holes are transported to the anode 32 by the hole transport material in the charge separation layer 33, and the electrons are transported to the cathode 34 by the electron transport material in the charge separation layer 33 and taken out to the external circuit.

When the benzobis(thiadiazole) derivative of the present invention, which has the high field-effect mobility, is used for the charge separation layer 33 of the organic PV device, holes and electrons may be efficiently taken out from the charge separation layer 33, and therefore the electricity generation efficiency may be enhanced. In addition, when the benzobis(thiadiazole) derivative of the present invention is used for the electron transport layer, electrons may be efficiently transported to the cathode, and therefore the electricity generation efficiency may be enhanced.

As the substrate 31, transparent materials such as glass and plastics may be used, for example. The charge separation layer 33, hole transport layer and electron transport layer may be formed by a coating method using the organic semiconductor ink of the present invention, and a flexible substrate may be used as the substrate 31 in similarly to the case of the organic TFT described above.

As the anode 32 as described above, a light-transmission material is generally used. Specifically, tin-doped indium oxide (ITO), indium oxide, tin oxide, indium oxide, and zinc oxide alloy are preferably used. A thin film of metal such as gold, platinum, silver, and magnesium alloy may also be used. In addition, organic materials such as polyaniline, polythiophene, polypyrrole, and derivatives thereof may also be used. The anode 32 may be formed by well-known film-formation methods such as vacuum deposition, electron-beam evaporation deposition, RF sputtering, and coating (printing).

As the cathode 34 as described above, alkali metals such as Li, K and Na, and alkali-earth metals such as Mg and Ca, which have small work function, are preferably used, from the viewpoint of the electron injection properties. In addition, Al which is stable, and the like are also preferably used. In order to achieve both stability and electron injection properties, the cathode may be a layer comprising two or more materials. The cathode 34 may be formed by well-known film-formation methods such as vacuum deposition, electron-beam evaporation deposition, and RF sputtering.

The benzobis(thiadiazole) derivative of the present invention as organic semiconductor materials is used for the charge separation layer 33 as described above. The benzobis(thiadiazole) derivative may be used singly, or may be used in combination of two or more. In addition, the charge separation layer 33 may comprise one or more of other organic semiconductor compounds.

Examples of the materials constituting the charge separation layer, together with the benzobis(thiadiazole) derivative, include poly(3-hexylthiophene-2,5-diyl) (P3HT) and poly[2-methoxy-5-(2-ethylhexyloxy)-1,4-phenylenevinylene] (MEH-PPV), as the hole transport material, and fullerene C60, (6,6)-phenyl-C61-butyric acid methyl ester (C61-PCBM), fullerene C70 and (6,6)-phenyl-C71-butyric acid methyl ester (C71-PCBM), as the electron transport material.

The charge separation layer 33 may be formed by well-known film-formation methods such as vacuum deposition, sputtering, and coating (printing). Preferably, the charge separation layer 33 may be formed by a coating (printing) method using the organic semiconductor ink of the present invention. The thickness of the charge separation layer 33 may be appropriately selected from the conventionally known range, and may be generally 5 nm to 1 µm, preferably 10 nm to 500 nm.

The organic PV device of the present invention may further comprise a hole transport layer and/or an electron transport layer. The benzobis(thiadiazole) derivative of the present invention may also be suitably used for these layers. The benzobis(thiadiazole) derivative may be used singly, or may be used in combination of two or more. In addition, the hole transport layer and the electron transport layer may comprise one or more of other compounds.

In the cases where the benzobis(thiadiazole) derivative of the present invention is not used for the hole transport layer or the electron transport layer, materials such as poly(3,4-ethylenedioxythiophene)-poly(styrenesulfonate) (PEDOT-PSS), for example, may be used as the hole transport layer, and materials such as 2,9-dimethyl-4,7-diphenyl-1,10-phenanthroline (BCP), for example, maybe used as the electron transport layer.

As the method of film-formation of the hole transport layer and the electron transport layer, the methods as listed for the method of film-formation of the charge separation layer 33 may be used. The thicknesses of the hole transport layer and the electron transport layer may be appropriately selected from the conventionally known range, and may be generally 1 nm to 1 µm, preferably 1 nm to 100 nm.

### (RFID tag)

The RFID tag (Radio Frequency Identification tag) of the present invention will be described below. The RFID tag of the present invention is activated by the use of an organic TFT, and the organic TFT is the organic TFT of the present invention as described above.

The RFID tag is an electronic device comprising an integrated circuit section (hereinafter, referred to as "IC section") and an antenna section, wherein the ID, i.e. personal identification information, or the like, stored in the IC section can be wirelessly communicated with a reader-writer device via the antenna section, and the information stored in the IC section can be read out and conversely information can be written to the IC section by the reader-writer device in a non-contact manner.

Article information management, distribution management, individual authentication, and the like can be performed when an RFID tag is stuck on an article or printed on an article, and an RFID tag is applied to various fields. The responsiveness of an RFID tag may be enhanced when the benzobis(thiadiazole) derivative of the present invention is used for the organic semiconductor layer of the organic TFT constituting the IC section of the RFID tag, because the high field-effect mobility may be achieved.

The RFID tag of the present invention may be produced by combinating any known circuits, which is described in Solid-State Electronics 84 (2013) 167-178, for example.

Any known structure and any known material may be used for the RFID tag of the present invention, except that the organic TFT of the present invention is employed. The organic TFT may be produced by any known method.

The IC section of the RFID tag may comprises a plurality of organic TFTs. In that case, among a plurality of organic TFTs, all of the organic TFTs may be the organic TFTs of the present invention, or alternatively, a part of the organic TFTs may be the organic TFTs of the present invention.

### (Sensor)

The sensor of the present invention will be described below. The sensor of the present invention is activated by the use of an organic TFT, and the organic TFT is the organic TFT of the present invention as described above.

The sensor may be applied to a system wherein the physical state of the organic semiconductor constituting the organic TFT is changed by an external stimulus applied to the organic TFT, and thereby the electric current and/or the electric voltage derived from the organic TFT are changed.

Examples of the sensor of the present invention include, for example, a temperature sensor and a light sensor, which detect the temperature or the light by the change of the physical state of the organic semiconductor constituting the organic TFT when the organic semiconductor is subjected to heat or light.

Examples thereof also include a bending-stress sensor and a pressure sensor, which detect the stress or the pressure by the change of the physical state of the organic semiconductor constituting the organic TFT when the organic semiconductor is subjected to stress.

Examples thereof also include a chemical sensor, which detects a certain chemical substance, for example, water molecule, oxygen molecule, or the like, by the change of the physical state of the organic semiconductor constituting the organic TFT when the organic semiconductor is influenced by the chemical substance.

Additionally, examples thereof also include a chemical sensor, which detects a certain chemical substance by the change of the electric current and/or the electric voltage derived from the organic TFT which occurs when the metal electrode constituting the organic TFT is influenced by the chemical substance.

The responsiveness or the dynamic range of the sensor may be enhanced when the benzobis(thiadiazole) derivative of the present invention is used for the semiconductor layer of the organic TFT constituting the sensor, because the high field-effect mobility may be achieved.

Any known structure and any known material may be used for the sensor of the present invention, except that the organic TFT of the present invention is employed. The sensor may be produced by any known method.

The sensor may comprise a single organic TFT, or may comprise a plurality of organic TFTs, which depends on the intended use. In the case where the sensor comprises a plurality of organic TFTs, among the organic TFTs, all of the organic TFTs may be the organic TFTs of the present invention, or alternatively, a part of the organic TFTs may be the organic TFTs of the present invention.

In the various organic electronic devices of the present invention as described above, a plastic substrate may be used as the substrate. The plastic to be used as the substrate may preferably have excellent heat resistance, dimensional stability, solvent resistance, electrical insulation property, processability, low air permeability, and low hygroscopicity. Examples of the plastic include, for example, polyethylene terephthalate, polyethylene naphthalate, polystyrene, polycarbonate, polyacrylate, and polyimide.

In the case of plastic substrate, it is preferred that a moisture-permeation blocking layer (gas barrier layer) be formed on the electrode side of the substrate, or the side opposite to the electrode, or on both sides. As the material constituting the moisture-permeation blocking layer, inorganic materials such as silicon nitride and silicon oxide are preferably used. The moisture-permeation blocking layer may be formed by well-known film-formation methods such as RF sputtering. In addition, a hard-coating layer or an undercoating layer may be formed as necessary.

In addition, for the purpose of adjusting the surface energy of the substrate or controlling the affinity with materials to be laminated on the substrate, the substrate in the organic electronic device of the present invention may be subjected to a substrate surface modification treatment, for example, the substrate may be treated with hexamethyldisilazane or octyltrichlorosilane, coated with a resin such as polystyrene, subjected to a UV ozone treatment, or the like.

Also, in addition to the benzobis(thiadiazole) derivative of the present invention represented by formulae (1) and (2) as described above, derivatives represented by the following formulae are also useful as the organic semiconductor materials.

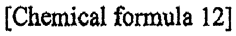

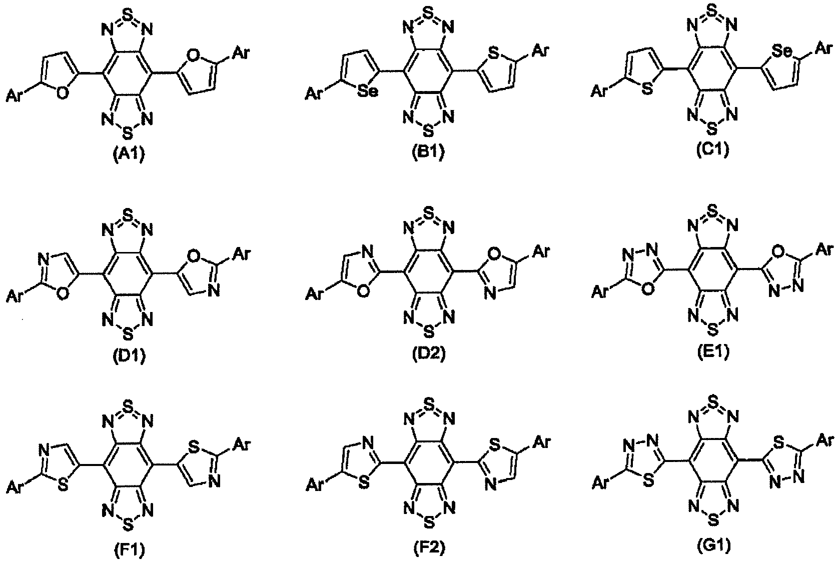

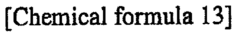

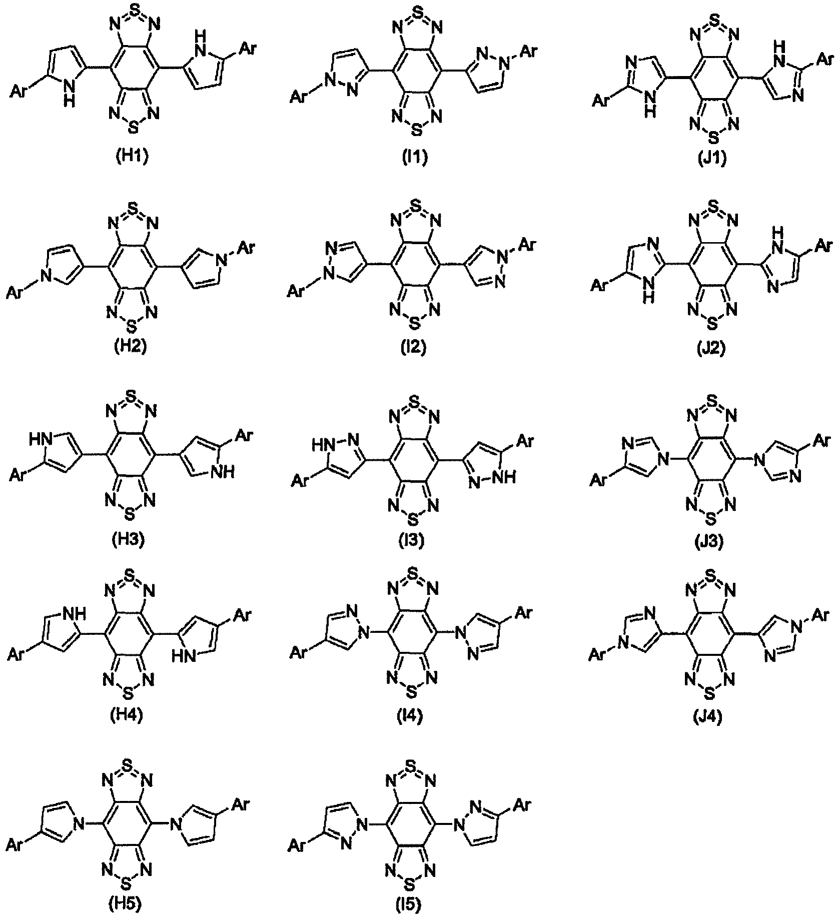

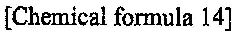

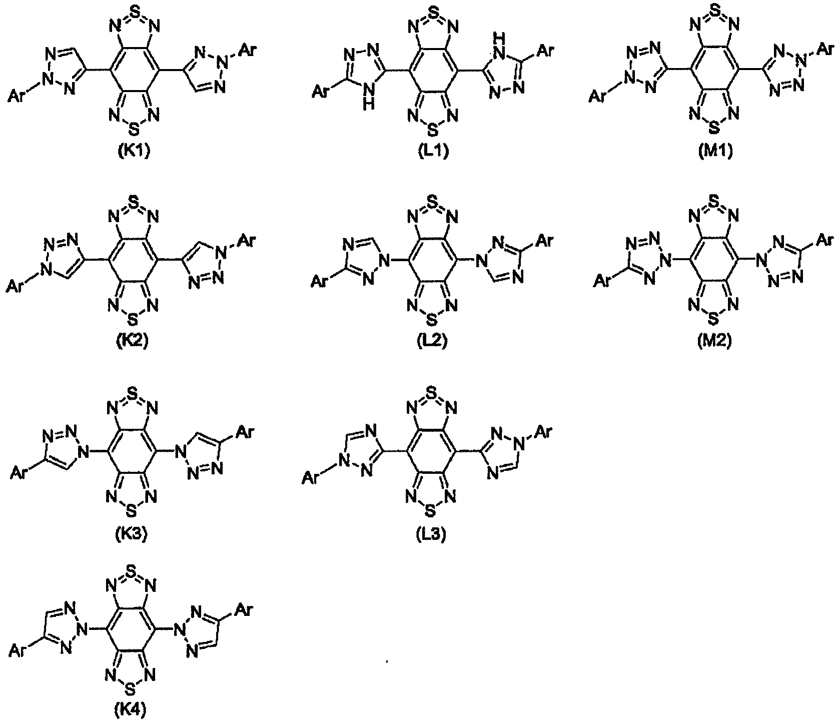

Wherein, Ar represents aryl group (there are two Ars for each compound, and these may be same or different), preferably a group represented by the following formulae.

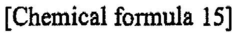

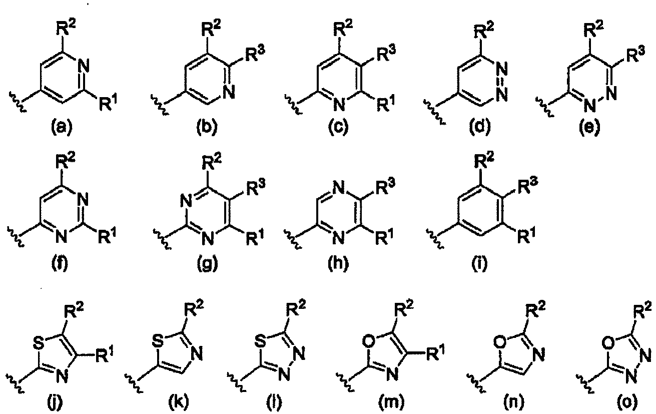

Wherein, a wavy line represent a bond position to the benzobis(thiadiazole) skeleton represented by (A1) to (M2). Also, R¹, R² and R³ are each independently a hydrogen atom, alkyl group, or the following group.

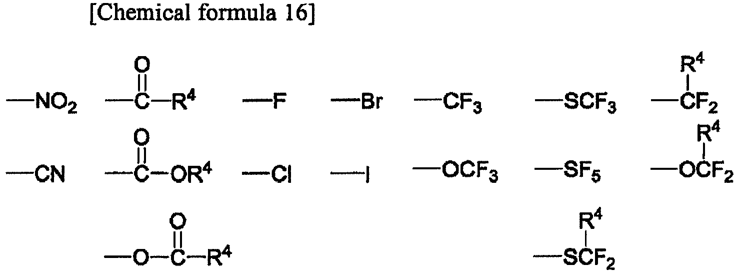

Wherein, R⁴ represents a linear or branched alkyl group. A hydrogen atom in the alkyl group can be substituted by a fluorine atom.

For the linear or branched alkyl group, from the viewpoint of the improvement in field-effect mobility and solubility of the benzobis(thiadiazole) derivative represented by (A1) to (M2), the carbon number of the alkyl group is preferably 1 to 30 , more preferably 3 to 28, particularly preferably 5 to 25. Also, for the branched alkyl group, the branched chain moiety and the main chain moiety may be bounded each other to form the cyclic structure. That is, the branched alkyl groups include cycloalkyl group.

Specific examples of the linear alkyl group include, for example, methyl group, ethyl group, propyl group, butyl group, pentyl group, hexyl group, heptyl group, octyl group, nonyl group, decyl group, undecyl group, dodecyl group, tridecyl group, tetradecyl group, pentadecyl group, hexadecyl group, heptadecyl group, and octadecyl group. A hydrogen atom on these alkyl groups can be substituted by a fluorine atom.

Specific examples of the branched alkyl group include, for example, isopropyl group, 1-methylpropyl group, 1-methylbutyl group, 2-methylhexyl group, 2-ethylhexyl group, 3-methylhexyl group, 3-ethylhexyl group, 2-methyloctyl group, 2-ethyloctyl group, 2-hexyldecyl group, 2-octyldodecyl group, 2-decyltetradecyl group, 3-methyloctyl group, and 3-ethyloctyl group. A hydrogen atom on these alkyl groups can be substituted by a fluorine atom.

### Examples

The present invention will be specifically described below with reference to the Examples. However, the present invention should not be limited to these Examples.

[Evaluation of Solubility] The evaluation of the solubility of the synthesized compound was conducted by the following method.

About 2-3 mg of the synthesized compound was precisely weighed, and each solvent was added to the compound such that a predetermined concentration of the solute (0.3 wt%, 0.2 wt%, 0.1 wt%) was reached. The resultant mixture was stirred for 30 minutes at a predetermined temperature, and then it was evaluated by visual appearance observation whether or not the solute (synthesized compound) was completely dissolved in the solvent, which was the result of the evaluation of the solubility. As the solvent, chloroform (boiling point 61°C), toluene (boiling point 111°C), anisole (boiling point 154°C), mesitylene (boiling point 165°C), ortho-dichlorobenzene (boiling point 181°C) and 1-methylnaphthalene (boiling point 241°C) were used.

[Production/Evaluation of organic TFT]In the Examples described below, a bottom gate-top contact device was produced on a silicon substrate in accordance with "Procedure for Producing organic TFT" as described below and the evaluation of the device was conducted, unless otherwise described in each section.

### <Procedure for producing organic TFT>

In the case where the production of the organic semiconductor layer by spin-coating method was carried out therein, an organic TFT was produced in accordance with the following procedure:
(Production of TFT substrate) - (Production of organic semiconductor layer by spin-coating method) - (Production of source electrode and drain electrode)
   or
(Production of TFT substrate) - (Surface modification of TFT substrate) -(Production of organic semiconductor layer by spin-coating method) - (Production of source electrode and drain electrode).

In the case where the production of the organic semiconductor layer by vacuum deposition method was carried out therein, an organic TFT was produced in accordance with the following procedure:
(Production of TFT substrate) - (Production of organic semiconductor layer by vacuum deposition method) - (Production of source electrode and drain electrode) or
(Production of TFT substrate) - (Surface modification of TFT substrate) -(Production of organic semiconductor layer by vacuum deposition method) - (Production of source electrode and drain electrode).

### (Production of TFT substrate)

A commercially available silicon wafer having a thermally grown silicon oxide with a film thickness of 200 nm formed on the surface was used as the substrate for the organic TFT. The silicon wafer had low resistance, and also functioned as the gate electrode of the organic TFT. In addition, the silicon oxide film was used as the gate insulating film. The silicon wafer was washed with a mixture solution of hydrogen peroxide water and sulfuric acid, and the surface was cleaned by UV ozone treatment immediately before the silicon wafer was used in the subsequent step. The substrate thus treated is referred to as "bare substrate" hereinafter.

### (Surface modification of TFT substrate)

The "bare substrate" was immersed and left still in hexamethyldisilazane, which was commercially available, for 12 hours or more, so that the surface of the substrate was modified. The substrate thus treated is referred to as "HMDS-modified substrate" hereinafter.

A solution was prepared by dissolving 5 mmol/L octyltrichlorosilane, which was commercially available, in toluene, and the "bare substrate" was immersed and left still in this solution for 12 hours or more to modify the substrate. The substrate thus treated is referred to as "OTS-modified substrate" hereinafter.

A solution prepared by dissolving 0.5 wt% polystyrene, which was commercially available, in xylene was applied onto the "bare substrate" by spin-coating, and then heated at 150°C for 1 hour, so that a polystyrene thin film with a thickness of about 20 nm was formed on the surface of the substrate. The substrate thus treated is referred to as "PS substrate" hereinafter.

A mixture solution of polyvinylphenol and melamine in propylene glycol monomethyl ether acetate as the solvent, which was prepared from polyvinylphenol and melamine, which were commercially available, was applied onto the "bare substrate" by spin-coating, and then heated at 180°C for 1 hour, so that a polyvinylphenol-melamine thin film with a thickness of about 20 nm was formed on the surface of the substrate. The substrate thus treated is referred to as "PVP substrate" hereinafter.

### (Production of organic semiconductor layer by spin-coating method)

With the use of the synthesized compound (organic semiconductor compound), a solution (organic semiconductor ink) with the solvent and the solute concentration described in each section was prepared, and with the use of the organic semiconductor ink, an organic semiconductor layer with a thickness of about 20-50 nm was formed on the substrate described in each section by spin-coating. And then, the organic semiconductor layer was subjected to thermal annealing under the conditions described in each section, as necessary. The spin-coating and the thermal annealing were performed in nitrogen atmosphere, unless otherwise described in each section.

### (Production of organic semiconductor layer by vacuum deposition)

With the use of the synthesized compound (organic semiconductor compound), an organic semiconductor layer with a thickness of about 50 nm was formed on the substrate described in each section by vacuum deposition. During the formation of the organic semiconductor layer, the pressure in the chamber of the vapor deposition apparatus was 2 x 10⁻⁵ - 6 × 10⁻⁴ Pa, and the organic semiconductor compound was contained in a crucible and heated by a filament wound around the crucible to perform vapor deposition. The deposition rate was 0.2 ± 0.1 Å/sec.

### (Production of source electrode and drain electrode)

A gold film was formed on the organic semiconductor layer by vacuum deposition, using a metal mask to form a source electrode and a drain electrode. The channel width and the channel length of the organic TFT were 1000 µm and 70 µm, respectively. The thickness of the source electrode and drain electrode was about 50 nm.

### (Evaluation of field-effect mobility)

The field-effect mobility (µ) was determined from the result of the measurement of the transfer characteristics of the produced organic TFT using a semiconductor characterization system, Model 4200-SCS from KEITHLEY Inc.

The field-effect mobility (µ) can be calculated using the following formula (Formula A), which represents the drain current I_{d}.

I_{d} = (W/2L)µCᵢ(V_{g}-Vₜ)² ... (Formula A)

Wherein L and W represent the channel length and the channel width, respectively, Cᵢ represents the capacity of the gate insulating layer per unit area, V_{g} represents the gate voltage, and Vₜ represents the threshold voltage. The measurement of the transfer characteristics reveals the threshold voltage and the drain current at a certain gate voltage, and therefore the field-effect mobility can be determined therefrom.

### <Example S-1>

### [Synthesis of Compound (12-6)]

According to the following synthetic scheme, Compound (12-6) was synthesized which is the benzobis(thiadiazole) derivative of the present invention. Each step will be specifically described below.

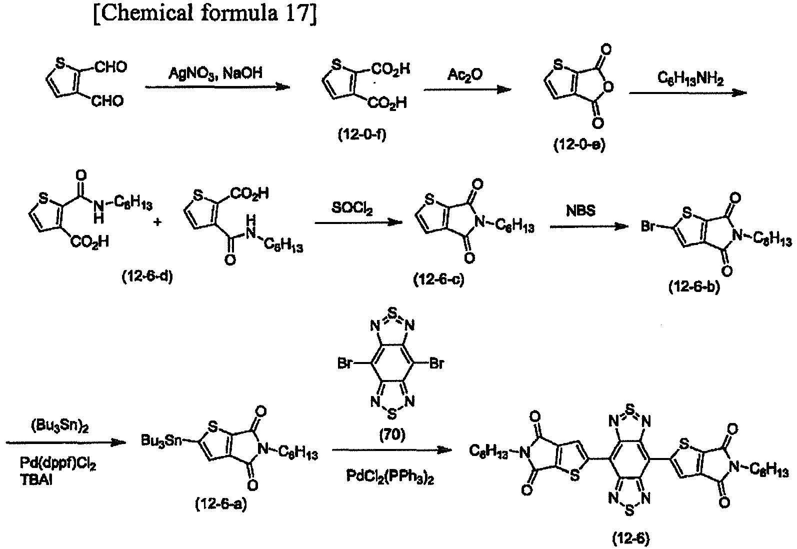

### (Step S1-1: Synthesis of Compound (12-0-f))

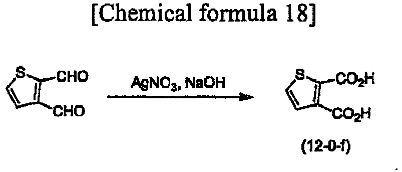

Into a 1 L glass reaction vessel equipped with a stirring apparatus were added 34.8 g (205.0 mmol) of silver nitrate and 100 mL of water to give a solution. Into this solution was added a solution of 16.4 g (410.0 mmol) of sodium hydroxide in 205 mL of water and stirred at room temperature for 15 minutes. Then, a solution of 7.0 g (50.0 mmol) of 2,3-thiophenedicarboxyaldehyde in 250 mL of ethanol was added dropwise over 1.5 hours.

After stirring at room temperature for 2 hours, the solution was suction filtered, and the resulting filtrate was concentrated and cooled with ice water. Then a 2 mol/L solution of hydrochloric acid in water was added thereto to give pH 1. The precipitate was suction filtered and dried under a reduced pressure to obtain 7.4 g of Compound (12-0-f) as a pale yellow ochre powder.

The properties of Compound (12-0-f) were as follows.
¹H-NMR (400 MHz; (CD₃)₂CO; δ(ppm)); 7.72 (d, 1H), 7.94 (d, 1H).
EI-MS; 172 (M+), CI-MS; 173 (M+1).

### (Step S1-2: Synthesis of Compound (12-0-e))

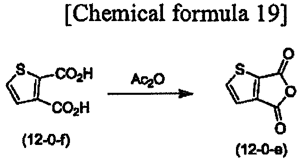

Into a 250 mL glass reaction vessel equipped with a stirring apparatus were added 6.9 g (40.0 mmol) of Compound (12-0-f) and 160 mL of acetic anhydride. The temperature of the mixture was increased from room temperature to 135°C and then the mixture was reacted at 135°C for 16 hours. After distilling off acetic anhydride under a reduced pressure, the resulting product was dissolved by adding toluene and heating at 65°C and an insoluble matter was filtered off. Then, hexane was added thereto and the deposited precipitate was filtered to obtain 5.6 g of Compound (12-0-e) as a pale brown solid.

The properties of Compound (12-0-e) were as follows.
¹H-NMR (400 MHz; (CD₃)₂CO; δ(ppm)); 7.58 (d, 1H), 8.43 (d, 1H).
EI-MS; 154 (M+).

### (Step S1-3: Synthesis of Compound (12-6-d))

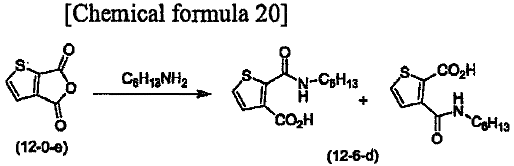

Into a 250 mL glass reaction vessel equipped with a stirring apparatus were added 1.7 g (11.0 mmol) of Compound (12-0-e) and 164 mL of toluene. The mixture was cooled with ice to 5°C, 1.5 mL (11.5 mmol) of normal hexylamine was added, and the mixture was reacted at room temperature for 4 hours. After distilling off toluene under a reduced pressure, diethyl ether added thereto and the precipitate was filtered to obtain 2.6 g of Compound (12-6-d) as a white solid.

The properties of Compound (12-6-d) were as follows.
¹H-NMR (400 MHz; CDCl₃; δ(ppm)); Major product: 0.86-0.92 (m, 3H), 1.29-1.43 (m, 6H), 1.62-1.77 (m, 2H), 3.45-3.52 (m, 2H), 6.74 (s, 1H), 7.33 (d, 1H), 7.58 (d, 1H).
Minor product: 0.86-0.92 (m, 3H), 1.29-1.43 (m, 6H), 1.62-1.77 (m, 2H), 3.45-3.52 (m, 2H), 7.10 (s, 1H), 7.40 (d, 1H), 7.79 (s, 1H).
EI-MS; 255 (M+), CI-MS; 256 (M+1).

Compound (12-6-d) was used for the next step without separation and purification.

### (Step S1-4: Synthesis of Compound (12-6-c))

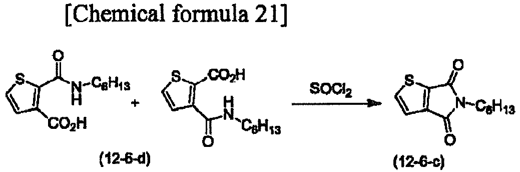

Into a 50 mL glass reaction vessel equipped with a stirring apparatus were added 255.3 mg (1.0 mmol) of Compound (12-6-d) and 19.7 mL of thionyl chloride. The temperature of the mixture was increased from room temperature to 90°C and then the mixture was reacted at 90°C for 3 hours. After distilling off thionyl chloride under a reduced pressure, the residue was purified by silica gel column chromatography (hexane: methylene chloride: ethyl acetate = 5: 1: 1) to obtain 218.4 mg of Compound (12-6-c) as a pale yellow solid.

The properties of Compound (12-6-c) were as follows.
¹H-NMR (400 MHz; CD₂Cl₂; δ(ppm)); 0.84-0.92 (m, 3H), 1.26-1.34 (m, 6H), 1.49-1.66 (m, 2H), 3.52-3.61 (m, 2H), 7.29 (d, 1H), 7.78 (d, 1H).
EI-MS; 237 (M+).

### (Step S1-5: Synthesis of Compound (12-6-b))

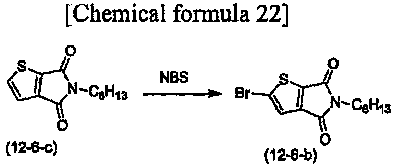

Into a 100 mL glass reaction vessel equipped with a stirring apparatus were added 1.8 g (7.6 mmol) of Compound (12-6-c), 45 mL of trifluoroacetic acid, 15 mL of sulfuric acid and 1.48 g (8.34 mmol) of N-bromosuccinimide, and the mixture was reacted at room temperature for 3 hours. Water was added to the reaction and the mixture was subjected to extraction with methylene chloride, and then the extract was dried over magnesium sulfate and the solvent was distilled off under a reduced pressure. The residue was purified by silica gel column chromatography (hexane: ethyl acetate = 10 : 1) to obtain 2.3 g of Compound (12-6-b) as a pale yellow solid.

In a series of steps (Step S1-1 to S1-7) in the synthesis of Compound (12-6), a plurality of experiments having different reaction scales were performed (similar for synthesis of Compound (22-6), Compound (22-1), Compound (12-8), Compound (12-EH), Compound (12-12), Compound (12-HepFBu), and Compound (12-Me) described below). For step S1-5, the reaction scale, which different from those corresponding to the experiment described for Step S1-4 described above, was described.

The properties of Compound (12-6-b) were as follows.
¹H-NMR (400 MHz; CD₂Cl₂; δ(ppm)); 0.86-0.90 (m, 3H), 1.27-1.33 (m, 6H), 1.57-1.62 (m, 2H), 3.51-3.57 (m, 2H), 7.31 (s, 1H).
EI-MS; 317 (M+).

### (Step S1-6: Synthesis of Compound (12-6-a))

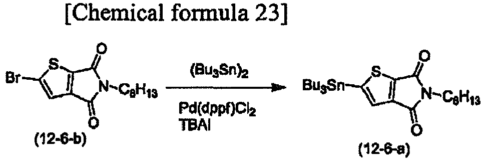

Into a 15 mL glass reaction vessel equipped with a stirring apparatus were added 158.1 mg (0.5 mmol) of Compound (12-6-b), 738.7 mg (2.0 mmol) of tetrabutylammonium iodide, 1.0 mL (2.0 mmol) of bistributyltin, 40.8 mg (0.05 mmol) of 1,1'-bis(diphenylphosphino)ferrocene palladium (II) dichloride-dichloromethane complex and 5 mL of 1,4-dioxane, and the mixture was reacted at 70°C for 4 hours. After distilling off 1,4-dioxane under a reduced pressure, the residue was purified by aminopropyl group-modified silica gel column chromatography (hexane) to obtain 34.2 mg of Compound (12-6-a) as a colorless liquid.

The properties of Compound (12-6-a) were as follows.
¹H-NMR (400 MHz; (CD₂Cl₂; δ(ppm)); 0.83-0.94 (m, 12H), 1.10-1.39 (m, 20H), 1.54-1.63 (m, 6H), 3.52-3.56 (m, 2H), 7.30 (s, 1H).
FD-MS; 526 (M+).

### (Step S1-7: Synthesis of Compound (12-6))

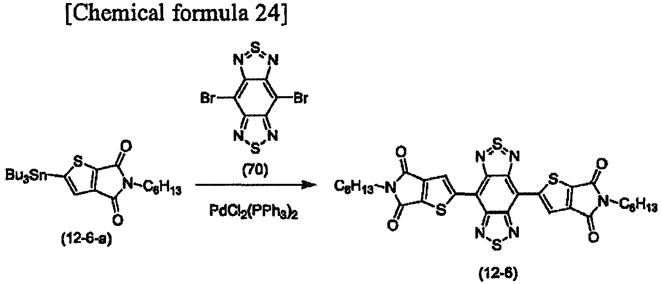

Into a 100 mL glass reaction vessel equipped with a stirring apparatus were added 3.0 g (5.7 mmol) of Compound (12-6-a), 503.4 mg (1.4 mmol) of dibromobenzobisthiadiazole (Compound (70)), 301.1 mg (0.4 mmol) of dichlorobis(triphenylphosphine) palladium (II) and 29 mL of dry toluene, and the mixture was reacted at temperature inside the reaction vessel of about 100°C for 6 hours. After distilling off toluene under a reduced pressure, the residue was purified by silica gel column chromatography (methylene chloride) to obtain 309.9 mg of Compound (12-6) as a dark green solid.

For step S 1-7, the reaction scale, which different from those corresponding to the experiment described for step S1-6 described above, was described.

The properties of Compound (12-6) were as follows.
¹H-NMR (400 MHz; CD₂Cl₂; δ(ppm)); 0.89-0.92 (m, 6H), 1.33-1.40 (m, 12H), 1.65-1.73 (m, 4H), 3.63-3.67 (m, 4H), 9.23 (s, 2H).
FD-MS; 664 (M+).

Also, the result of the thermal weight loss analysis showed that the initial decomposition temperature of Compound (12-6) was 364°C, and the result of differential scanning calorimetry analysis showed that Compound (12-6) had the melting point of 286°C and had not a peak due to a phase transition and the like from room temperature to melting point. As the result thereof, it was found that Compound (12-6) was a very thermally stable compound.

The solubility of Compound (12-6) was evaluated, and 0.2 wt% Compound (12-6) was completely dissolved in chloroform at room temperature (25°C). Also, 0.3 wt% Compound (12-6) was completely dissolved in mesitylene at 80°C. Also, 0.3 wt% Compound (12-6) was completely dissolved in toluene at 80°C. Also, 0.1 wt% Compound (12-6) was completely dissolved in 1-methylnaphthalene at room temperature (25°C).

### <Example S-2>

[Synthesis of Compound (22-6)] According to the following synthetic scheme, Compound (22-6) was synthesized which is the benzobis(thiadiazole) derivative of the present invention. Each step will be specifically described below.

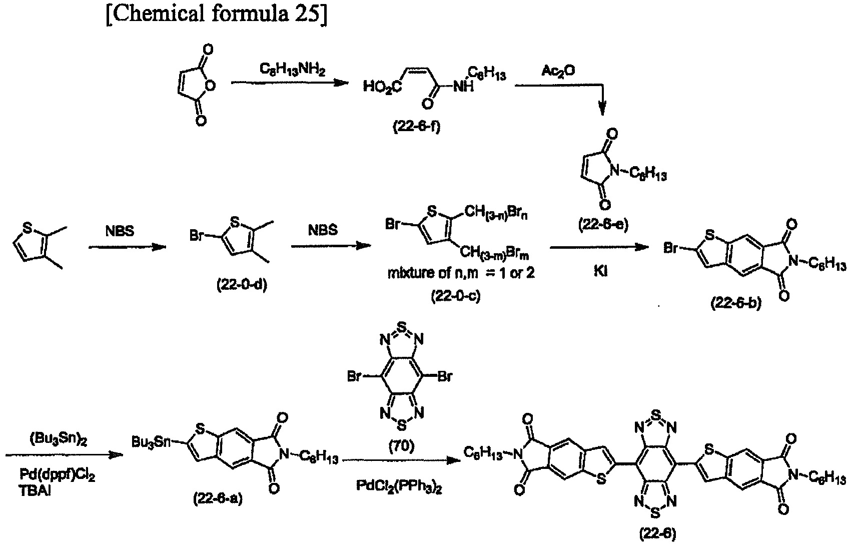

### (Step S2-1: Synthesis of Compound (22-6-f))

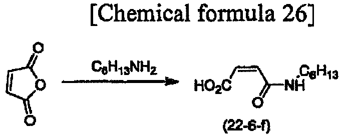

Into a 250 mL glass reaction vessel equipped with a stirring apparatus were added 2.5 g (25.0 mmol) of maleic anhydride and 100 mL of toluene, and the mixture was cooled to -70°C. Then, 3.3 mL (25.0 mmol) of normal hexylamine was added, the temperature was gradually increased to room temperature, and the mixture was reacted at room temperature for 12 hours. The deposited precipitate was suction filtered and washed with toluene to obtain 3.8 g of Compound (22-6-f) as a white solid.

The properties of Compound (22-6-f) were as follows.
¹H-NMR (400 MHz; CDCl₃; δ(ppm)); 0.85-0.93 (m, 3H), 1.27-1.39 (m, 6H), 1.56-1.79 (m, 2H), 3.29-3.43 (m, 2H), 6.32 (d, 1H), 6.40 (d, 1H), 7.36 (br, 1H).
CI-MS; 200 (M+1).

### (Step S2-2: Synthesis of Compound (22-6-e))

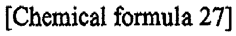

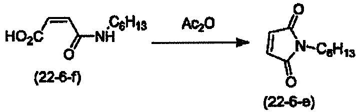

Into a 100 mL glass reaction vessel equipped with a stirring apparatus were added 3.6 g (18.0 mmol) of Compound (22-6-f), 0.74 g (9.0 mmol) of sodium acetate and 71 mL of acetic anhydride, and the mixture was reacted at 110°C for 4 hours. After distilling off acetic anhydride under a reduced pressure, the residue was purified by silica gel column chromatography (methylene chloride : hexane = 3 : 1) to obtain 2.4 g of Compound (22-6-e) as a white solid.

The properties of Compound (22-6-e) were as follows.
¹H-NMR (400 MHz; CDCl₃; δ(ppm)); 0.84-0.89 (m, 3H), 1.26-1.32 (m, 6H), 1.54-1.61 (m, 2H), 3.49-3.53 (m, 2H), 6.68 (s, 2H).
EI-MS; 181 (M+).

### (Step S2-3: Synthesis of Compound (22-0-d))

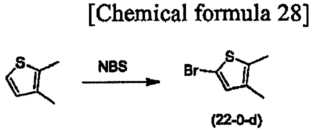

Into a 250 mL glass reaction vessel equipped with a stirring apparatus were added 2.2 mL (20.0 mmol) of dimethylthiophene and 100 mL of methylene chloride, and 3.9 g (22.0 mmol) of N-bromosuccinimide was added into the solution and then the mixture was reacted at room temperature for 15 hours. After distilling off methylene chloride under a reduced pressure, the residue was purified by silica gel column chromatography (hexane) to obtain 3.6 g of Compound (22-0-d) as a colorless liquid.

The properties of Compound (22-0-d) were as follows.
¹H-NMR (400 MHz; CD₂Cl₂; δ(ppm)); 2.08 (s, 3H), 2.26 (s, 3H), 6.73 (s, 1H).
EI-MS; 192 (M+).

### (Step S2-4: Synthesis of Compound (22-0-c))

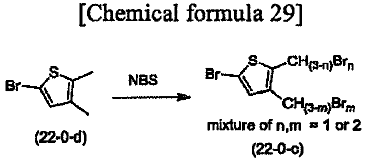

Into a 100 mL glass reaction vessel equipped with a stirring apparatus were added 0.96 g (5.0 mmol) of Compound (22-0-d) and 50 mL of carbon tetrachloride, and 3.9 g (22.0 mmol) of N-bromosuccinimide and 361.3 mg (2.2 mmol) of azobisisobutyronitrile were added into the solution and then the mixture was reacted at 85°C for 17 hours. Water was added to the reaction and the mixture was subjected to extraction with methylene chloride, and then the extract was dried over magnesium sulfate and the solvent was distilled off under a reduced pressure. Compound (22-0-c) was used for the next step without separation and purification.

### (Step S2-5: Synthesis of Compound (22-6-b))

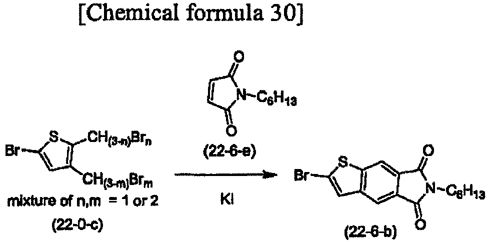

Into a 100 mL glass reaction vessel equipped with a stirring apparatus were added the whole quantity of Compound (22-0-c) obtained in step S2-4, 1.8 g (10.0 mmol) of Compound (22-6-e) obtained in step S2-2, 1.7 g (10.0 mmol) of potassium iodide and 30 mL of N,N-dimethylformamide, and the mixture was reacted at 140°C for 17 hours. After distilling off N,N-dimethylformamide under a reduced pressure, water was added to the reaction, and the mixture was subjected to extraction with methylene chloride, and then the extract was dried over magnesium sulfate and the solvent was distilled off under a reduced pressure.

The resulting dark brown solid was purified by silica gel column chromatography (methylene chloride: hexane = 1 : 1), and the resulting yellow solid was recrystallization from hexane to obtain 710.5 mg of Compound (22-6-b) as yellow crystals.

The properties of Compound (22-6-b) were as follows.
¹H-NMR (400 MHz; CD₂Cl₂; δ(ppm)); 0.86-0.90 (m, 3H), 1.28-1.38 (m, 6H), 1.63-1.71 (m, 2H), 3.63-3.69 (m, 2H), 7.58 (s, 1H), 8.12 (s, 1H), 8.18 (s, 1H).
EI-MS; 366 (M+).

### (Step S2-6: Synthesis of Compound (22-6-a))

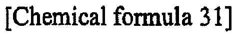

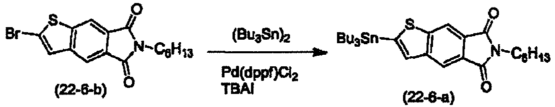

Compound (22-6-a) was obtained in the same way as in step S1-6, except that Compound (22-6-b) was used instead of Compound (12-6-b) of step S1-6.

The properties of Compound (22-6-a) were as follows.
¹H-NMR (400 MHz; CD₂Cl₂; δ(ppm)); 0.89-0.92 (m, 12H), 1.21-1.41 (m, 20H), 1.55-1.69 (m, 6H), 3.65-3.69 (m, 2H), 7.61 (s, 1H), 8.20 (s, 1H), 8.29 (s, 1H).
FD-MS; 576 (M+).

### (Step S2-7: Synthesis of Compound (22-6))

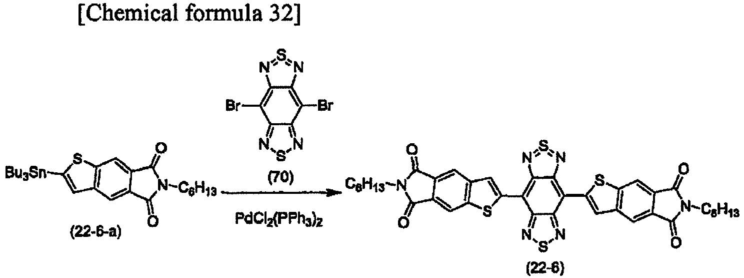

Compound (22-6) was obtained in the same way as in step S1-7, except that Compound (22-6-a) was used instead of Compound (12-6-a) of step S1-7.

The properties of Compound (22-6) were as follows.
¹H-NMR (400 MHz; C₆D₄Cl₂: 100°C; δ(ppm)); 1.05-1.08 (m, 6H), 1.46-1.60 (m, 12H), 1.94-2.02 (m, 4H), 3.95-3.99 (m, 4H), 8.49 (s, 4H), 9.71 (s, 2H).
FD-MS; 764 (M+).

Also, the result of the thermal weight loss analysis showed that the initial decomposition temperature of Compound (22-6) was 405°C, and the result of differential scanning calorimetry analysis showed that Compound (22-6) had not a peak due to a phase transition and the like at temperature up to initial decomposition temperature including melting point. As the result thereof, it was found that Compound (22-6) was a very thermally stable compound.

### <Example S-3>

### [Synthesis of Compound (22-1)]

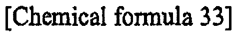

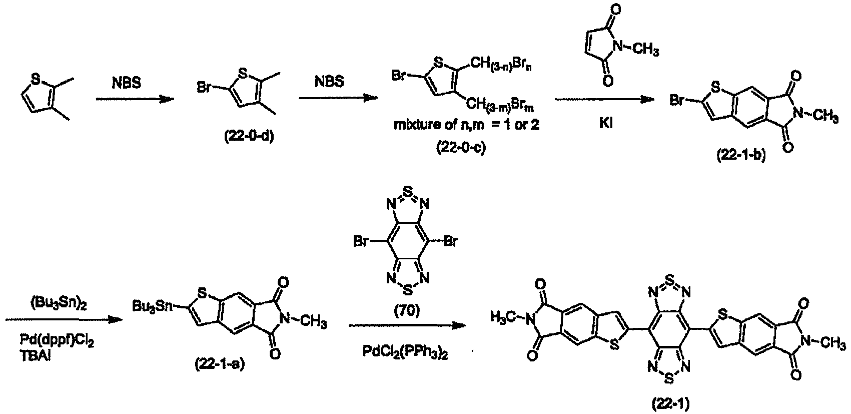

### (Step S3-1: Synthesis of Compound (22-1-b))

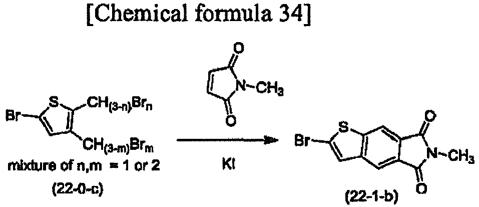

Compound (22-1-b) was obtained in the same way as in step S2-5, except that a commercially available N-methylmaleimide was used instead of Compound (22-6-e) of step S2-5.

The properties of Compound (22-1-b) were as follows.
¹H-NMR (400 MHz; CD₂Cl₂; δ(ppm)); 3.17 (s, 3H), 7.58 (s, 1H), 8.13 (s, 1H), 8.19 (s, 1H).
EI-MS; 297 (M+).

### (Step S3-2: Synthesis of Compound (22-1-a))

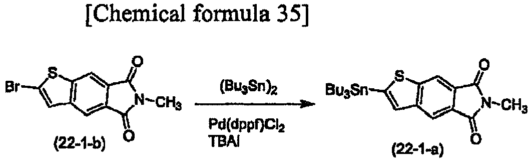

Compound (22-1-a) was obtained in the same way as in step S1-6, except that Compound (22-1-b) was used instead of Compound (12-6-b) of step S1-6.

The properties of Compound (22-1-a) were as follows.
¹H-NMR (400 MHz; CD₂Cl₂; δ(ppm)); 0.89-0.92 (m, 9H), 1.21-1.25 (m, 6H), 1.32-1.41 (m, 6H), 1.58-1.66 (m, 6H), 3.16 (s, 3H), 7.61 (s, 1H), 8.21 (s, 1H), 8.30 (s, 1H). FD-MS; 506 (M+).

### (Step S3-3: Synthesis of Compound (22-1))

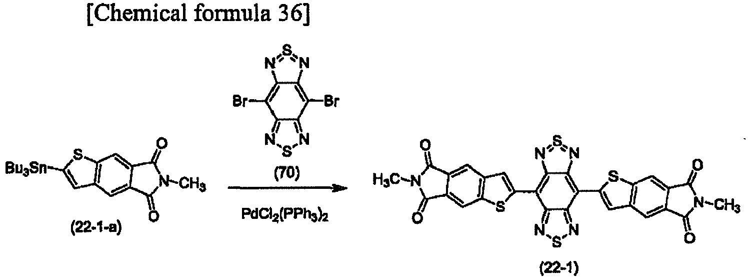

Compound (22-1) was obtained in the same way as in step S1-7, except that Compound (22-1-a) was used instead of Compound (12-6-a) of step S1-7.

The properties of Compound (22-1) were as follows.
FD-MS; 624 (M+).

Also, the result of the thermal weight loss analysis showed that the initial decomposition temperature of Compound (22-1) was 475°C, and the result of differential scanning calorimetry analysis showed that Compound (22-1) had not a peak due to a phase transition and the like at temperature up to initial decomposition temperature including melting point. As the result thereof, it was found that Compound (22-1) was a very thermally stable compound.

### <Example S-4>

### [Synthesis of Compound (12-8)]

Step S1-3 of the synthesis of Compound (12-6) shown in Example S-1: Compound (12-8) was obtained in the same step as in Example S-1, except that normal octylamine was used instead of normal hexylamine used in the synthesis of Compound (12-6-d).

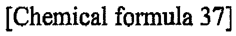

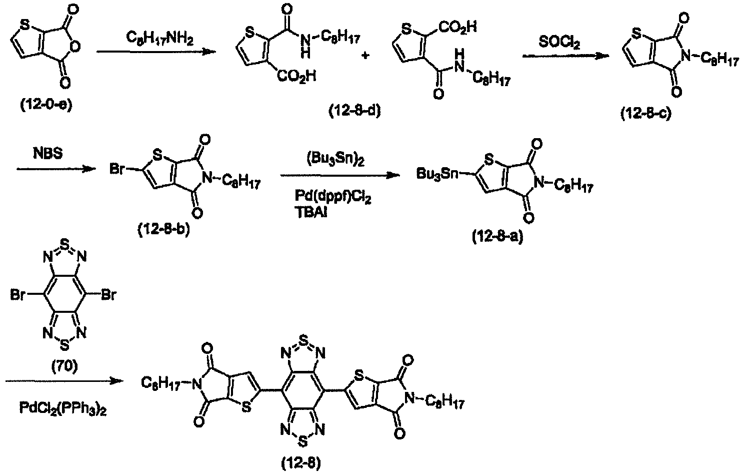

### (Step S4-1: Synthesis of Compound (12-8-d))

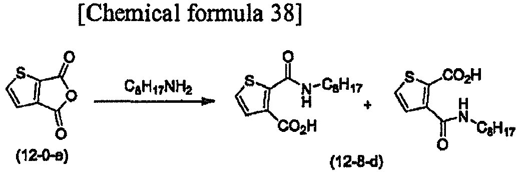

Into a 500 mL glass reaction vessel equipped with a stirring apparatus were added 3.9 g (25.0 mmol) of Compound (12-0-e) and 250 mL of toluene. The mixture was cooled with ice to 5°C, 4.5 mL (27.5 mmol) of normal octylamine was added, and the mixture was reacted at room temperature for 15 hours. After distilling off toluene under a reduced pressure, normal hexane was added thereto and the precipitate was filtered to obtain 6.8 g of Compound (12-8-d) as a pale yellow powder.

The properties of Compound (12-8-d) were as follows. ¹H-NMR (400 MHz; (CD₃)₂CO; δ(ppm)); Major product: 0.84-0.89 (m, 3H), 1.28-1.44 (m, 10H), 1.58-1.70 (m, 2H), 3.46-3.48 (m, 2H), 7.64 (s, 1H), 7.75 (d, 1H), 7.82 (d, 1H). Minor product: 0.86-0.89 (m, 3H), 1.28-1.42 (m, 10H), 1.60-1.69 (m, 2H), 3.39-3.45 (m, 2H), 7.66 (d, 1H), 7.72 (d, 1H), 9.43 (br, 1H).
EI-MS; 283 (M+), CI-MS; 284 (M+1).

Compound (12-8-d) was used for the next step without separation and purification.

### (Step S4-2: Synthesis of Compound (12-8-c))

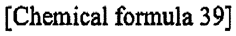

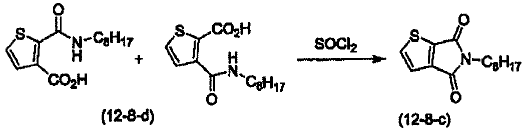

Into a 300 mL glass reaction vessel equipped with a stirring apparatus were added 4.3 g (15.2 mmol) of Compound (12-8-d) and 166 mL of thionyl chloride. The temperature of the mixture was increased from room temperature to 90°C and the mixture was reacted at 90°C for 17 hours. After distilling off thionyl chloride under a reduced pressure, the residue was purified by silica gel column chromatography to obtain 3.7 g of Compound (12-8-c) as a pale yellow solid.

The properties of Compound (12-8-c) were as follows.
¹H-NMR (400 MHz; CD₂Cl₂; δ(ppm)); 0.85-0.89 (m, 3H), 1.27-1.31 (m, 10H), 1.59-1.64 (m, 2H), 3.55-3.58 (m, 2H), 7.29 (d, 1H), 7.78 (d, 1H).
EI-MS; 265 (M+).

### (Step S4-3: Synthesis of Compound (12-8-b))

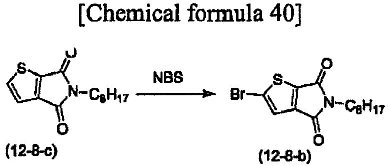

Into a 250 mL glass reaction vessel equipped with a stirring apparatus were added 4.7 g (17.5 mmol) of Compound (12-8-c), 105 mL of trifluoroacetic acid, 35 mL of sulfuric acid and 3.4 g (19.3 mmol) of N-bromosuccinimide, and the mixture was reacted at room temperature for 3 hours. Water was added to the reaction and the mixture was subjected to extraction with methylene chloride, and then the extract was dried over magnesium sulfate and the solvent was distilled off under a reduced pressure. The residue was purified by silica gel column chromatography to obtain 5.6 g of Compound (12-8-b) as a white solid.

For the step S4-3, the reaction scale, which different from those corresponding to the experiment described for step S4-2 described above, was described.

The properties of Compound (12-8-b) were as follows.
¹H-NMR (400 MHz; CD₂Cl₂; δ(ppm)); 0.85-0.91 (m, 3H), 1.23-1.30 (m, 10H), 1.58-1.62 (m, 2H), 3.53-3.59 (m, 2H), 7.32 (s, 1H).
EI-MS; 345 (M+).

### (Step S4-4: Synthesis of Compound (12-8-a))

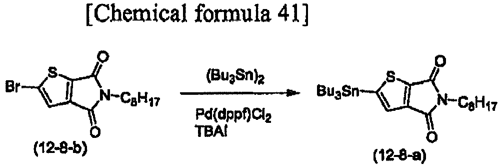

Into a 250 mL glass reaction vessel equipped with a stirring apparatus were added 5.8 g (17.0 mmol) of Compound (12-8-b), 12.5 g (33.9 mmol) of tetrabutylammonium iodide, 17.1 mL (33.9 mmol) of bistributyltin, 1.4 g (1.7 mmol) of 1,1'-bis(diphenylphosphino)ferrocene palladium (II) dichloride-dichloromethane complex and 170 mL of 1,4-dioxane, and the mixture was reacted at 70°C for 20 hours. After distilling off 1,4-dioxane under a reduced pressure, the mixture was extracted with normal hexane to obtain 5.2 g of Compound (12-8-a) as a dark brown liquid.

For step S4-4, the reaction scale, which different from those corresponding to the experiment described for step S4-3 described above, was described.

The properties of Compound (12-8-a) were as follows.
¹H-NMR (400 MHz; CD₂Cl₂; δ(ppm)); 0.87-0.93 (m, 12H), 1.17-1.39 (m, 20H), 1.55-1.63 (m, 10H), 3.52-3.56 (m, 2H), 7.32 (t, 1H).
FD-MS; 554 (M+).

### (Step S4-5: Synthesis of Compound (12-8))

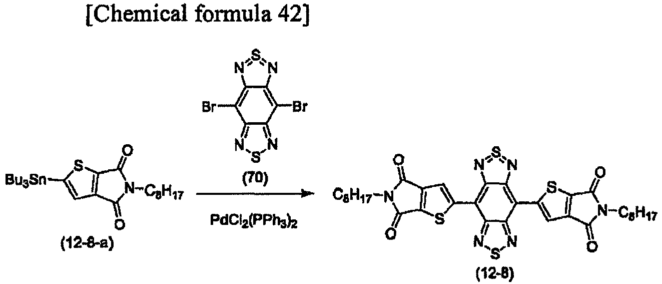

Into a 100 mL glass reaction vessel equipped with a stirring apparatus were added 4.9 g (8.8 mmol) of Compound (12-8-a), 770.9 mg (2.2 mmol) of dibromobenzobisthiadiazole (Compound (70)), 461.1 mg (0.7 mmol) of dichlorobis(triphenylphosphine) palladium (II) and 33 mL of dry toluene, and the mixture was reacted at temperature inside the reaction vessel of about 100°C for 7 hours. After distilling off toluene under a reduced pressure, the residue was purified by silica gel column chromatography and recrystallized from a mixed solvent of chloroform : methanol to obtain 194.3 mg of Compound (12-8) as a dark green solid.

The properties of Compound (12-8) were as follows.
¹H-NMR (400 MHz; CD₂Cl₂; δ(ppm)); 0.87-0.90 (m, 6H), 1.29-1.37 (m, 20H), 1.67-1.71 (m, 4H), 3.62-3.65 (m, 4H), 9.14 (s, 2H).
FD-MS; 720 (M+).

Also, the result of the thermal weight loss analysis showed that the initial decomposition temperature of Compound (12-8) was 367°C, and the result of differential scanning calorimetry analysis showed that Compound (12-8) had the melting point of 276°C. As the result thereof, it was found that Compound (12-8) was a very thermally stable compound.

The solubility of Compound (12-8) was evaluated, and 0.2 wt% Compound (12-8) was completely dissolved in chloroform at room temperature (25°C). Also, 0.3 wt% Compound (12-8) was completely dissolved in toluene at room temperature (25°C). Also, 0.3 wt% Compound (12-8) was completely dissolved in mesitylene at room temperature (25°C). Also, 0.3 wt% Compound (12-8) was completely dissolved in 1-methylnaphthalene at room temperature (25°C).

### <Example S-5>

### [Synthesis of Compound (12-EH)]

Step S1-3 of the synthesis of Compound (12-6) shown in Example S-1: Compound (12-EH) was obtained in the same step as in Example S-1, except that 2-ethylhexylamine was used instead of normal hexylamine used in the synthesis of Compound (12-6-d).

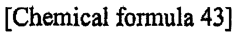

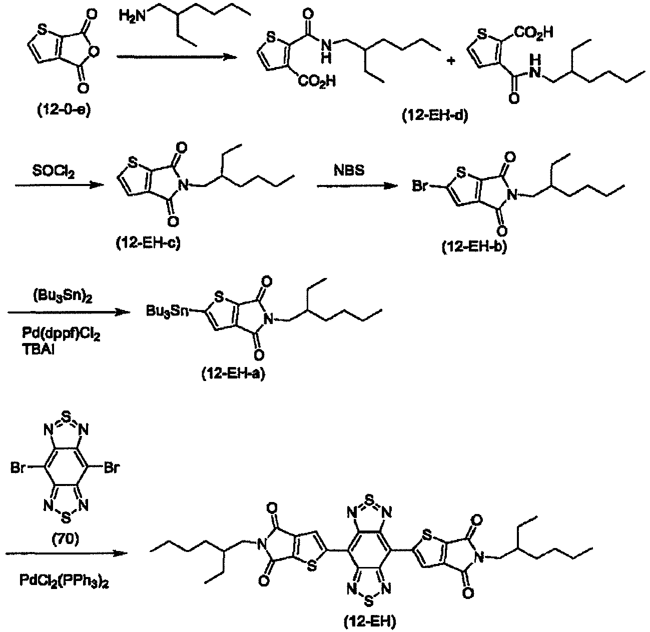

### (Step S5-1: Synthesis of Compound (12-EH-d))

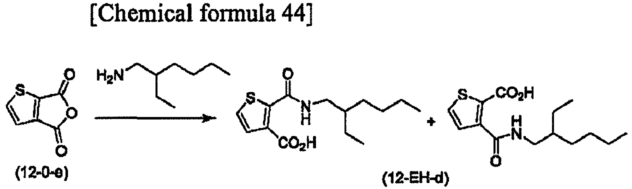

Into a 500 mL glass reaction vessel equipped with a stirring apparatus were added 4.6 g (30.0 mmol) of Compound (12-0-e) and 300 mL of toluene. The mixture was cooled with ice to 5°C, 5.4 mL (33.0 mmol) of 2-ethylhexylamine was added, and the mixture was reacted at room temperature for 5 hours. After distilling off toluene under a reduced pressure, normal hexane was added thereto and the precipitate was filtered to obtain 8.0 g of Compound (12-EH-d) as a white solid.

The properties of Compound (12-EH-d) were as follows.
¹H-NMR (400 MHz; (CD₃)₂CO; δ(ppm)); Major product: 0.91-0.93 (m, 6H), 1.31-1.36 (m, 8H), 1.64-1.69 (m, 1H), 3.39-3.41 (d, 2H) 7.62-7.65 (m, 1H), 7.75 (d, 1H), 7.79 (d, 1H). Minor product: 0.87-0.90 (m, 6H), 1.38-1.44 (m, 8H), 1.57-1.60 (m, 1H), 3.70 (d, 2H), 7.62-7.65 (m, 1H), 7.75 (d, 1H), 7.79 (d, 1H).
EI-MS; 283 (M+), CI-MS; 284 (M+1).

Compound (12-EH-d) was used for the next step without separation and purification.

### (Step S5-2: Synthesis of Compound (12-EH-c))

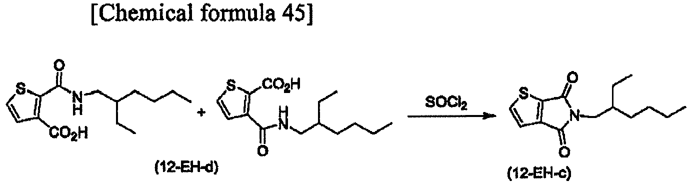

Into a 500 mL glass reaction vessel equipped with a stirring apparatus were added 8.5 g (30.0 mmol) of Compound (12-EH-d) and 328 mL of thionyl chloride. The temperature of the mixture was increased from room temperature to 90°C, and the mixture was reacted at 90°C for 16 hours. After distilling off thionyl chloride under a reduced pressure, the residue was purified by silica gel column chromatography to obtain 7.6 g of Compound (12-EH-c) as a pale yellow viscous liquid.

For step S5-2, the reaction scale, which different from those corresponding to the experiment described for step S5-1 described above, was described.

The properties of Compound (12-EH-c) were as follows.
¹H-NMR (400 MHz; CD₂Cl₂; δ(ppm)); 0.88-0.92 (m, 6H), 1.23-1.39 (m, 8H), 1.72-1.81 (m, 1H), 3.47 (d, 2H), 7.30 (d, 1H), 7.78 (d, 1H).
EI-MS; 265 (M+).

### (Step S5-3: Synthesis of Compound (12-EH-b))

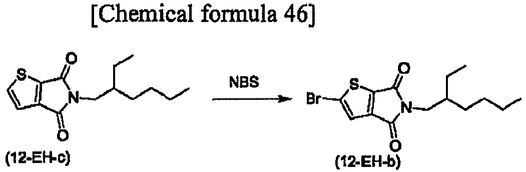

Into a 250 mL glass reaction vessel equipped with a stirring apparatus were added 4.9 g (18.6 mmol) of Compound (12-EH-c), 111 mL of trifluoroacetic acid, 37 mL of sulfuric acid and 3.6 g (20.4 mmol) of N-bromosuccinimide, and the mixture was reacted at room temperature for 3 hours. Water was added to the reaction and the mixture was subjected to extraction with methylene chloride, and then the extract was dried over magnesium sulfate and the solvent was distilled off under a reduced pressure. The residue was purified by silica gel column chromatography to obtain 6.1 g of Compound (12-EH-b) as a white solid.

The properties of Compound (12-EH-b) were as follows.
¹H-NMR (400 MHz; (CD₂Cl₂; δ(ppm)); 0.87-0.91 (m, 6H), 1.22-1.39 (m, 8H), 1.71-1.77 (m, 1H), 3.45 (d, 2H), 7.32 (s, 1H).
EI-MS; 345 (M+).

### (Step S5-4: Synthesis of Compound (12-EH-a))

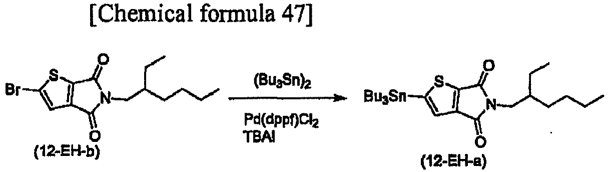

Into a 250 mL glass reaction vessel equipped with a stirring apparatus were added 6.9 g (20.0 mmol) of Compound (12-EH-b), 14.8 g (40.0 mmol) of tetrabutylammonium iodide, 20.2 mL (40.0 mmol) of bistributyltin, 1.63 g (2.0 mmol) of 1,1'-bis(diphenylphosphino)ferrocene palladium (II) dichloride-dichloromethane complex and 200 mL of 1,4-dioxane, and the mixture was reacted at 70°C for 24 hours. After distilling off 1,4-dioxane under a reduced pressure, the mixture was extracted with normal hexane to obtain 6.1g of Compound (12-EH-a) as a dark brown liquid.

For step S5-4, the reaction scale, which different from those corresponding to the experiment described for step S5-3 described above, was described.

The properties of Compound (12-EH-a) were as follows.
¹H-NMR (400 MHz; (CD₂Cl₂; δ(ppm)); 0.87-1.00 (m, 15H), 1.27-1.65 (m, 27H), 3.44 (d, 2H), 7.30 (t, 1H).
FD-MS; 554 (M+).

### (Step S5-5: Synthesis of Compound (12-EH))

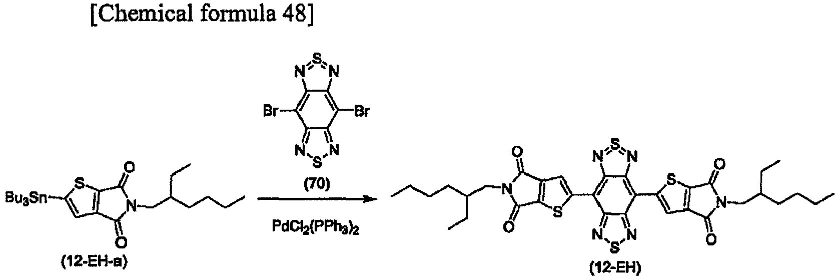

Into a 100 mL glass reaction vessel equipped with a stirring apparatus were added 6.8 g (12.3 mmol) of Compound (12-EH-a), 1.1 g (3.1 mmol) of dibromobenzobisthiadiazole (Compound (70)), 646.4 mg (0.9 mmol) of dichlorobis(triphenylphosphine) palladium (II) and 46 mL of dry toluene, and the mixture was reacted at temperature inside the reaction vessel of about 100°C for 7 hours. After distilling off toluene under a reduced pressure, the residue was purified by silica gel column chromatography and recrystallized from a mixed solvent of chloroform : methanol to obtain 186.0 mg of Compound (12-EH) as a dark green solid.

For step S5-5, the reaction scale, which different from those corresponding to the experiment described for step S5-4 described above, was described.

The properties of Compound (12-EH) were as follows.
¹H-NMR (400 MHz; CD₂Cl₂; δ(ppm)); 0.89-0.96 (m, 12H), 1.32-1.46 (m, 16H), 1.82-1.85 (m, 2H), 3.55 (d, 4H), 9.23 (s, 2H).
FD-MS; 720 (M+).

Also, the result of the thermal weight loss analysis showed that the initial decomposition temperature of Compound (12-EH) was 369°C, and the result of differential scanning calorimetry analysis showed that Compound (12-EH) had the melting point of 315°C. As the result thereof, it was found that Compound (12-EH) was a very thermally stable compound.

The solubility of Compound (12-EH) was evaluated, and 0.2 wt% Compound (12-EH) was completely dissolved in chloroform at room temperature (25°C). Also, 0.3 wt% Compound (12-EH) was completely dissolved in 1-methylnaphthalene at room temperature (25°C). Also, 0.3 wt% Compound (12-EH) was completely dissolved in ortho-dichlorobenzene at room temperature (25°C). Also, 0.3 wt% Compound (12-EH) was completely dissolved in toluene at 60°C. Also, 0.3 wt% Compound (12-EH) was completely dissolved in mesitylene at 80°C.

### <Example S-6>

### [Synthesis of Compound (12-12)]

Step S 1-3 of the synthesis of Compound (12-6) shown in Example S-1: Compound (12-12) was obtained in the same step as in Example S-1, except that normal dodecylamine was used instead of normal hexylamine used in the synthesis of Compound (12-6-d).

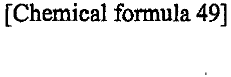

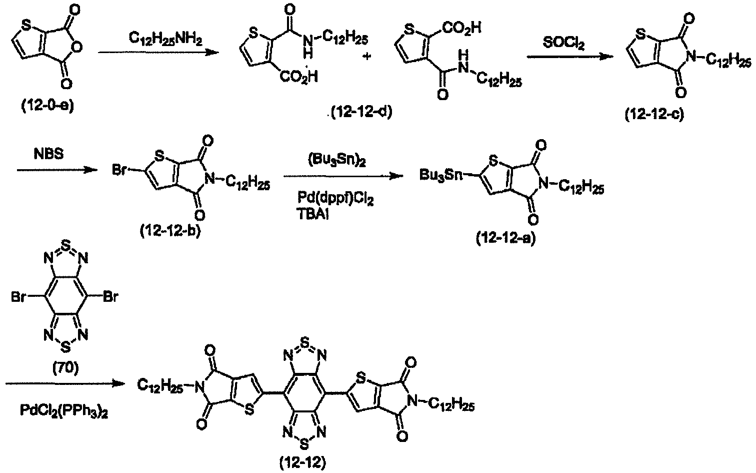

### (Step S6-1: Synthesis of Compound (12-12-d))

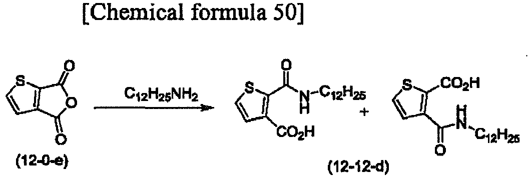

Into a 500 mL glass reaction vessel equipped with a stirring apparatus were added 4.6 g (30.0 mmol) of Compound (12-0-e) and 300 mL of toluene. The mixture was cooled with ice to 5°C, 6.1 g (33.0 mmol) of normal dodecylamine was added, and the mixture was reacted at room temperature for 22 hours. After distilling off toluene under a reduced pressure, normal hexane was added thereto and the precipitate was filtered to obtain 10.0 g of Compound (12-12-d) as a pale gray powder.

The properties of Compound (12-12-d) were as follows.
¹H-NMR (400 MHz; (CD₃)₂SO; δ(ppm)); Major product: 0.83-0.87 (m, 3H), 1.24-1.29 (m, 18H), 1.47-1.54 (m, 2H), 3.23-3.28 (m, 2H), 7.47 (d, 1H), 7.59 (d, 1H), 10.25 (br, 1H). Minor product: 0.83-0.87 (m, 3H), 1.24-1.29 (m, 18H), 1.47-1.54 (m, 2H), 2.49-2.51 (m, 2H), 7.67 (s, 1H), 7.79 (d, 1H), 10.00 (br, 1H).
EI-MS; 339 (M+), CI-MS; 340 (M+1).

Compound (12-12-d) was used for the next step without separation and purification.

### (Step S6-2: Synthesis of Compound (12-12-c))

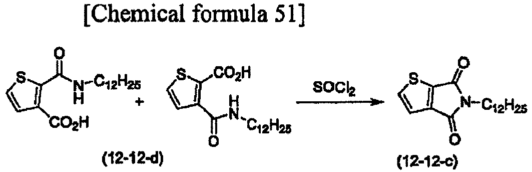

Into a 500 mL glass reaction vessel equipped with a stirring apparatus were added 9.9 g (29.0 mmol) of Compound (12-12-d) and 317 mL of thionyl chloride. The temperature of the mixture was increased from room temperature to 90°C, and the mixture was reacted at 90°C for 17 hours. After distilling off thionyl chloride under a reduced pressure, the residue was purified by silica gel column chromatography to obtain 8.1 g of Compound (12-12-c) as a pale brown solid.

The properties of Compound (12-12-c) were as follows.
¹H-NMR (400 MHz; CD₂Cl₂; δ(ppm)); 0.86-0.91 (m, 3H), 1.26-1.31 (m, 18H), 1.60-1.63 (m, 2H), 3.54-3.58 (m, 2H), 7.29 (d, 1H), 7.77 (d, 1H).
EI-MS; 321 (M+).

### (Step S6-3: Synthesis of Compound (12-12-b))

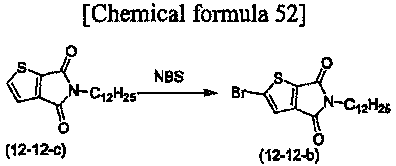

Into a 500 mL glass reaction vessel equipped with a stirring apparatus were added 8.0 g (25.0 mmol) of Compound (12-12-c), 150 mL of trifluoroacetic acid, 50 mL of sulfuric acid and 4.9 g (27.5 mmol) of N-bromosuccinimide, and the mixture was reacted at room temperature for 3 hours. Water was added to the reaction and the mixture was subjected to extraction with methylene chloride, and then the extract was dried over magnesium sulfate and the solvent was distilled off under a reduced pressure. The residue was purified by silica gel column chromatography to obtain 9.4 g of Compound (12-12-b) as a pale orange solid.

The properties of Compound (12-12-b) were as follows.
¹H-NMR 400 MHz; CD₂Cl₂; δ(ppm)); 0.86-0.89 (m, 3H), 1.26-1.30 (m, 18H), 1.59-1.64 (m, 2H), 3.51-3.59 (m, 2H), 7.31 (s, 1H).
EI-MS; 399 (M+).

### (Step S6-4: Synthesis of Compound (12-12-a))

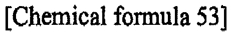

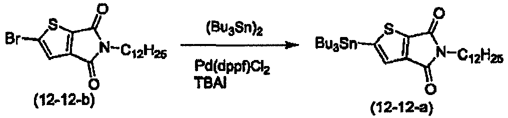

Into a 15 mL glass reaction vessel equipped with a stirring apparatus were added 9.4 g (23.5 mmol) of Compound (12-12-b), 17.4 mg (47.0 mmol) of tetrabutylammonium iodide, 23.8 mL (47.0 mmol) of bistributyltin, 1.9 g (2.4 mmol) of 1,1'-bis(diphenylphosphino)ferrocene palladium (II) dichloride-dichloromethane complex and 235 mL of 1,4-dioxane, and the mixture was reacted at 70°C for 19 hours. After distilling off 1,4-dioxane under a reduced pressure, the mixture was extracted with normal hexane to obtain 8.6 g of Compound (12-12-a) as a dark brown liquid.

The properties of Compound (12-12-a) were as follows.
¹H-NMR (400 MHz; CD₂Cl₂; δ(ppm)); 0.87-1.00 (m, 12H), 1.26-1.63 (m, 38H), 3.52-3.56 (m, 2H), 7.30 (t, 1H).
FD-MS; 610 (M+).

### (Step S6-5: Synthesis of Compound (12-12))

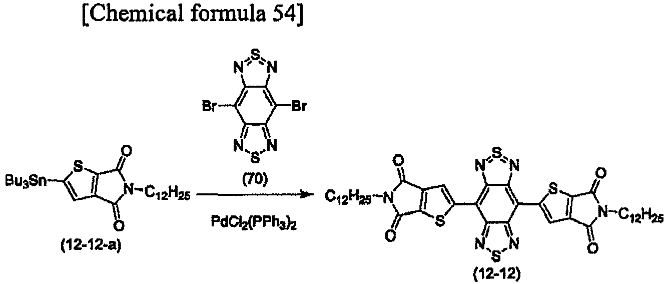

Into a 250 mL glass reaction vessel equipped with a stirring apparatus were added 8.6 g (14.0 mmol) of Compound (12-12-a), 1.23 g (3.5 mmol) of dibromobenzobisthiadiazole (Compound (70)), 737.0 mg (1.1 mmol) of dichlorobis(triphenylphosphine) palladium (II) and 53 mL of dry toluene, and the mixture was reacted at temperature inside the reaction vessel of about 100°C for 7 hours. After distilling off toluene under a reduced pressure, the residue was purified by silica gel column chromatography, and recrystallized from a mixed solvent of chloroform: methanol to obtain 47.5 mg of Compound (12-12) as a dark blue solid.

The properties of Compound (12-12) were as follows.
¹H-NMR (400 MHz; CDCl₃; δ(ppm)); 0.85-0.89 (m, 6H), 1.26-1.35 (m, 36H), 1.69-1.72 (m, 4H), 3.65-3.69 (m, 4H), 9.27 (s, 2H).
FD-MS; 833 (M+).

Also, the result of the thermal weight loss analysis showed that the initial decomposition temperature of Compound (12-12) was 360°C, and the result of differential scanning calorimetry analysis showed that Compound (12-12) had the melting point of 257°C. As the result thereof, it was found that Compound (12-12) was a very thermally stable compound.

The solubility of Compound (12-12) was evaluated, and 0.1 wt% Compound (12-12) was completely dissolved in chloroform at room temperature (25°C). Also, 0.3 wt% Compound (12-12) was completely dissolved in toluene at 60°C. Also, 0.3 wt% Compound (12-12) was completely dissolved in mesitylene at 60°C.

### <Example S-7>

### [Synthesis of Compound (12-HepFBu)]

According to the scheme described below, Compound (12-HepFBu) was obtained using compound (Compound (12-0-e)) shown in Example S-1.

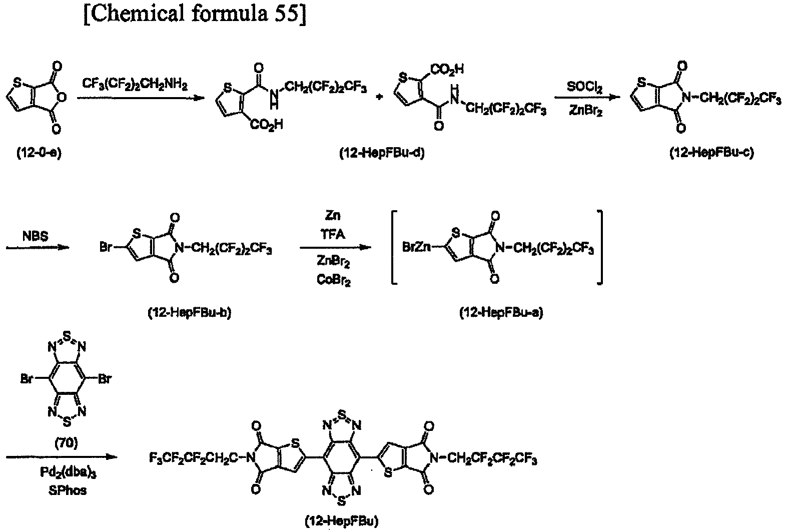

### (Step S7-1: Synthesis of Compound (12-HepFBu-d))

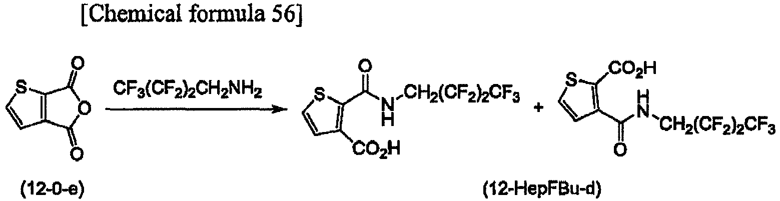

Into a 500 mL glass reaction vessel equipped with a stirring apparatus were added 3.9 g (25.0 mmol) of Compound (12-0-e) and 250 mL of toluene. The mixture was cooled with ice to 5°C, 4.0 mL (30.0 mmol) of 2,2,3,3,4,4,4-heptafluorobutylamine was added, and the mixture was reacted at room temperature for 16 hours. After distilling off toluene under a reduced pressure, normal hexane was added thereto and the precipitate was filtered to obtain 8.7 g of Compound (12-HepFBu-d) as a pale yellow ochre powder.

The properties of Compound (12-HepFBu-d) were as follows.
¹H-NMR (400 MHz; (CD₃)₂CO; δ(ppm)); Major product: 4.30-4.41 (m, 2H), 7.78 (d, 1H), 7.79 (d, 1H). Minor product: 3.91-3.99 (m, 2H), 1.28-1.42 (m, 10H), 1.60-1.69 (m, 2H), 7.70 (d, 1H), 7.83 (d, 1H).
EI-MS; 353 (M+), CI-MS; 354 (M+1).

Compound (12-HepFBu-d) was used for the next step without separation and purification.

### (Step S7-2: Synthesis of Compound (12-HepFBu-c))

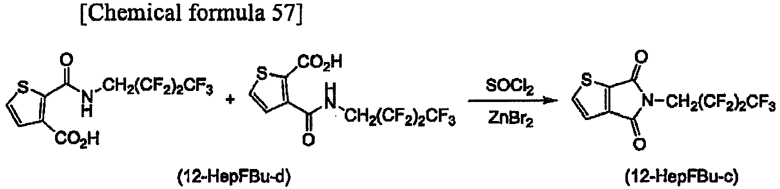

Into a 500 mL glass reaction vessel equipped with a stirring apparatus were added 8.4 g (23.8 mmol) of Compound (12-HepFBu-d), 1.6 g (7.1 mmol) of zinc bromide and 260 mL of thionyl chloride. The temperature of the mixture was increased from room temperature to 90°C, and the mixture was reacted at 90°C for 5 hours. After distilling off thionyl chloride under a reduced pressure, the residue was purified by silica gel column chromatography to obtain 7.1 g of Compound (12-HepFBu-c) as a white solid.

The properties of Compound (12-HepFBu-c) were as follows.
¹H-NMR (400 MHz; CD₂Cl₂; δ(ppm)); 4.25-4.33 (m, 2H), 7.38 (d, 1H), 7.89 (d, 1H). EI-MS; 335 (M+).

### (Step S7-3: Synthesis of Compound (12-HepFBu-b))

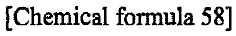

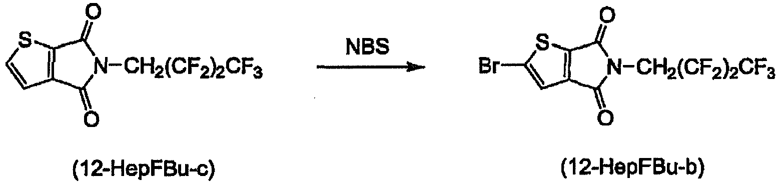

Into a 300 mL glass reaction vessel equipped with a stirring apparatus were added 8.2 g (24.5 mmol) of Compound (12-HepFBu-c), 146 mL of trifluoroacetic acid, 49 mL of sulfuric acid and 4.8 g (26.7 mmol) of N-bromosuccinimide, and the mixture was reacted at room temperature for 3 hours. Water was added to the reaction and the mixture was subjected to extraction with methylene chloride, and then the extract was dried over magnesium sulfate and the solvent was distilled off under a reduced pressure. The residue was purified by silica gel column chromatography to obtain 9.7 g of Compound (12-HepFBu-b) as a white solid.

For step S7-3, the reaction scale, which different from those corresponding to the experiment described for step S7-2 described above, was described.

The properties of Compound (12-HepFBu-b) were as follows.
¹H-NMR (400 MHz; CD₂Cl₂; δ(ppm)); 4.23-4.33 (m, 2H), 7.41 (s, 1H).
EI-MS; 415 (M+).

### (Step S7-4: Synthesis of Compound (12-HepFBu))

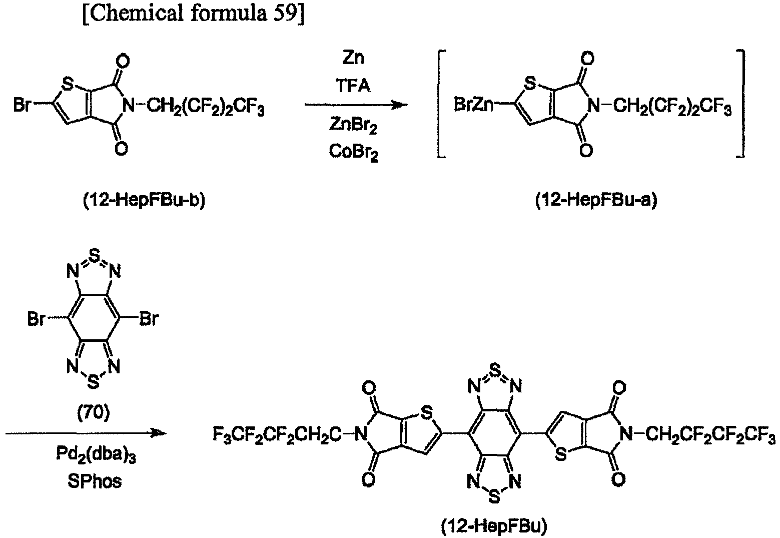

Into a 50 mL glass reaction vessel equipped with a stirring apparatus were added 784.6 mg (12.0 mmol) of zinc powder compound, 20 mL of acetonitrile and 18 µL (0.24 mmol) of trifluoroacetic acid. After stirring at room temperature for 15 minutes, 1.7 g (4.0 mmol) of Compound (12-HepFBu-b) was added. The mixture was cooled to -30°C, 90.1 mg (0.4 mmol) of zinc bromide and 175.0 mg (0.8 mmol) of cobalt bromide were added thereto, and then the mixture was stirred at 0°C for 1 hour. After distilling off acetonitrile under a reduced pressure, 20 mL of tetrahydrofuran was added, and Compound (12-HepFBu-a) was extracted.

Then, into a 50 mL glass reaction vessel equipped with a stirring apparatus were added 91.6 mg (0.1 mmol) of tris(dibenzylideneacetone) palladium, 164.2 mg (0.4 mmol) of 2-dicyclohexylphosphino-2',6'-dimethoxybiphenyl (also referred to as "SPhos") and 5 mL of tetrahydrofuran, and the mixture was stirred at room temperature for 15 minutes. And then, 352.0 mg (1.0 mmol) of dibromobenzobisthiadiazole (Compound (70)) and the previously extracted Compound (12-HepFBu-a) solution were added, and the mixture was reacted at 50°C for 21.5 hours. After distilling off tetrahydrofuran under a reduced pressure, the residue was purified by silica gel column chromatography and recrystallized from a mixed solvent of toluene : methanol to obtain 26.1 mg of Compound (12-HepFBu) as a dark blue solid.

The properties of Compound (12-HepFBu) were as follows.
¹H-NMR (400 MHz;C₄D₈O; δ(ppm));4.46 (m, 4H), 9.32 (s, 2H).
FD-MS; 860 (M+).

Also, the result of the thermal weight loss analysis showed that the initial decomposition temperature of Compound (12-HepFBu) was 367°C, and the result of differential scanning calorimetry analysis showed that Compound (12-HepFBu) had the melting point of 276°C. As the result thereof, it was found that Compound (12-HepFBu) was a very thermally stable compound.

The solubility of Compound (12-HepFBu) was evaluated, and 0.1 wt% Compound (12-HepFBu) was completely dissolved in chloroform at room temperature (25°C). Also, 0.1 wt% Compound (12-HepFBu) was completely dissolved in mesitylene at room temperature (25°C). Also, 0.1 wt% Compound (12-HepFBu) was completely dissolved in anisole at room temperature (25°C).

### <Example S-8>

### [Synthesis of Compound (12-Me)]

According to the scheme described below, Compound (12-Me) was obtained using compound (Compound (12-0-e)) shown in Example S-1.

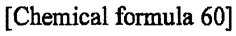

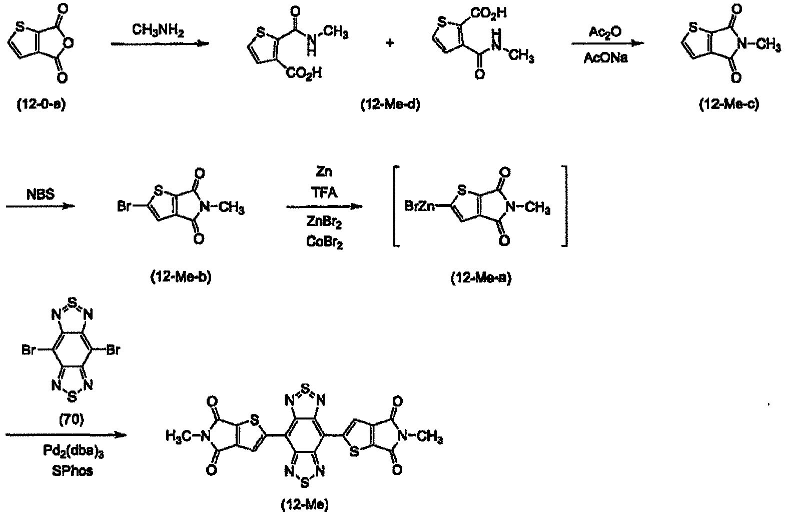

### (Step S8-1: Synthesis of Compound (12-Me-d))

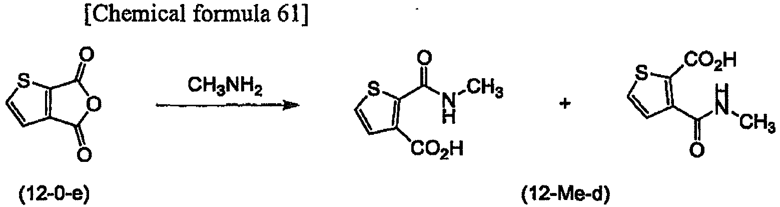

Into a 500 mL glass reaction vessel equipped with a stirring apparatus were added 4.6 g (30.0 mmol) of Compound (12-0-e) and 300 mL of toluene. The mixture was cooled with ice to 5°C, 16.5 mL (33.0 mmol) of a solution of methylamine in tetrahydrofuran (2M/L) was added dropwise over 15 minutes, and then the mixture was stirred at room temperature for 15 hours. After distilling off toluene under a reduced pressure, normal hexane was added thereto and the precipitate was filtered to obtain 5.5 g of Compound (12-Me-d) as a pale yellow ochre powder.

The properties of Compound (12-Me-d) were as follows.
¹H-NMR (400 MHz; (CD₃)₂CO; δ(ppm)); Major product: 2.99-3.00 (m, 3H), 7.72 (d, 1H), 7.87 (d, 1H), 8.71 (br, 1H).
Minor product: 2.95-2.96 (m, 2H), 7.66 (d, 1H), 7.71 (d, 1H), 9.22 (br, 1H).
EI-MS; 185 (M+), CI-MS; 186 (M+1).

Compound (12-Me-d) was used for the next step without separation and purification.

### (Step S8-2: Synthesis of Compound (12-Me-c))

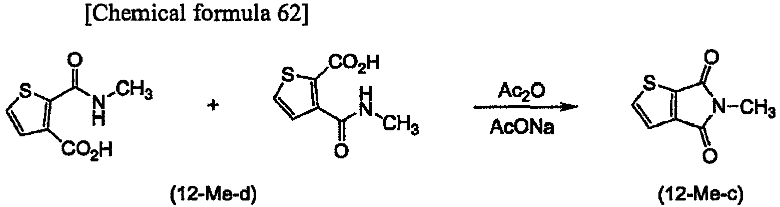

Into a 250 mL glass reaction vessel equipped with a stirring apparatus were added 3.3 g (18.0 mmol) of Compound (12-Me-d), 738.3 mg (9.0 mmol) of sodium acetate and 85 mL of acetic anhydride. The temperature of the mixture was increased from room temperature to 110°C, and the mixture was reacted at 110°C for 23 hours. After distilling off acetic anhydride under a reduced pressure, the residue was purified by silica gel column chromatography to obtain 2.5 g of Compound (12-Me-c) as a white solid.

The properties of Compound (12-Me-c) were as follows.
¹H-NMR (400 MHz; (CD₂Cl₂; δ(ppm)); 3.07 (s, 3H), 7.29 (d, 1H), 7.78 (d, 1H). EI-MS; 167 (M+).

### (Step S8-3: Synthesis of Compound (12-Me-b))

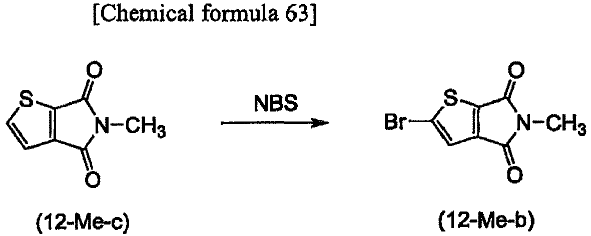

Into a 500 mL glass reaction vessel equipped with a stirring apparatus were added 3.8 g (22.7 mmol) of Compound (12-Me-c), 136 mL of trifluoroacetic acid, 46 mL of sulfuric acid and 4.5 g (25.0 mmol) of N-bromosuccinimide, and the mixture was reacted at room temperature for 3 hours. Water was added to the reaction and the mixture was subjected to extraction with methylene chloride, and then the extract was dried over magnesium sulfate and the solvent was distilled off under a reduced pressure. The residue was purified by silica gel column chromatography to obtain 4.7 g of Compound (12-Me-b) as a pale yellow solid.

For step S8-3, the reaction scale, which different from those corresponding to the experiment described for step S8-2 described above, was described.

The properties of Compound (12-Me-b) were as follows.
¹H-NMR (400 MHz; CD₂Cl₂; δ(ppm)); 3.06 (s, 3H), 7.33 (s, 1H). EI-MS; 247 (M+).

### (Step S8-4: Synthesis of Compound (12-Me))

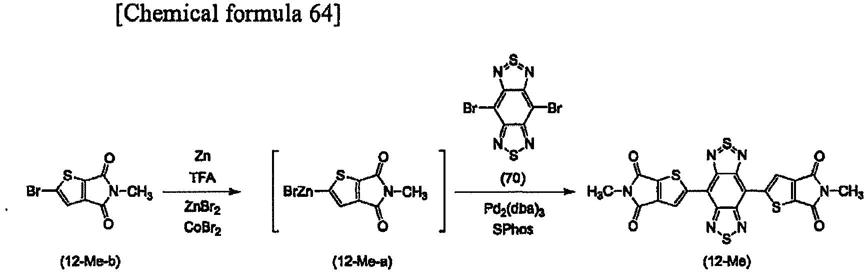

Into a 50 mL glass reaction vessel equipped with a stirring apparatus were added 588.4 mg (9.0 mmol) of zinc powder, 15 mL of acetonitrile and 14 µL (0.18 mmol) of trifluoroacetic acid, and the mixture was stirred at room temperature for 15 minutes. Then, 738.2 mg (3.0 mmol) of Compound (12-Me-b) was added, followed by 67.6 mg (0.3 mmol) of zinc bromide and 131.2 mg (0.6 mmol) of cobalt bromide, and the mixture was stirred at room temperature for 17 hours. After distilling off acetonitrile under a reduced pressure, 15 mL of tetrahydrofuran was added, and Compound (12-Me-a) was extracted.

Then, into a 50 mL glass reaction vessel equipped with a stirring apparatus were added 91.6 mg (0.1 mmol) of tris(dibenzylideneacetone)palladium, 164.2 mg (0.4 mmol) of 2-dicyclohexylphosphino-2',6'-dimethoxybiphenyl (SPhos) and 5 mL of tetrahydrofuran, and the mixture was stirred at room temperature for 15 minutes. Then, 352.0 mg (1.0 mmol) of dibromobenzobisthiadiazole (Compound (70)) and the previously extracted Compound (12-Me-a) solution were added, and the mixture was reacted at 50°C for 5 hours. After distilling off tetrahydrofuran under a reduced pressure, the residue was purified by silica gel column chromatography to obtain 26.2 mg of Compound (12-Me) as a dark blue solid.

The properties of Compound (12-Me) were as follows.
¹H-NMR (400 MHz; CDCl₃; δ(ppm)); 3.08 (s, 6H), 9.26 (s, 2H).
FD-MS; 524 (M+).

Also, the result of the thermal weight loss analysis showed that the initial decomposition temperature of Compound (12-me) was 367°C, and the result of differential scanning calorimetry analysis showed that Compound (12-Me) had the melting point of 276°C. As the result thereof, it was found that Compound (12-Me) was a very thermally stable compound.

### <Example E-1a>

With the use of the Compound (12-6) obtained in Example S-1, an organic TFT was produced by forming an organic semiconductor layer on a "bare substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-6) to chloroform such that the concentration was 0.1 wt% was dropped onto the "bare substrate" under an atmosphere of nitrogen, and then spin-coating was performed at 1000 rpm for 30 seconds, to form an organic semiconductor layer with a thickness of about 20 nm. And then, the "bare substrate" on which the organic semiconductor layer was formed was heated at 180°C for 35 minutes under an atmosphere of nitrogen.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics. The obtained transfer characteristics are shown in Fig. 6. In Fig. 6, the horizontal axis indicates gate voltage (V), and the vertical axis indicates drain current (A). The curve with gray-lacquered circle indicates fore, and the curve with outline character circle indicates back.

The field-effect mobility (µ) was calculated using the above-described (Formula A), and as the result thereof, it was found that the organic TFT comprising compound (12-6) on the "bare substrate" had a field-effect mobility of 3.9×10⁻¹ cm²/Vs.

### <Example E-1b>

An organic TFT was produced in the same way as in Example E-1a using the compound (12-6) obtained in Example S-1, except that the organic semiconductor ink which was prepared at 0.2 wt% in chloroform was used as the condition of production of organic semiconductor layer. The transfer characteristics of the produced organic TFT were measured under the same condition as in Example E-1a, and as the result thereof, it was found that the organic TFT comprising compound (12-6) on the "bare substrate" had a field-effect mobility of 3.6×10⁻¹ cm²/Vs.

After the organic TFT was left in the atmosphere for 28 days, the transfer characteristics of the produced organic TFT were measured under the same condition as in Example E-1a, and as the result thereof, it was found that a field-effect mobility of 3.9×10⁻¹ cm²/Vs is obtained. As the result thereof, it was found that the organic TFT of the present invention stable in the atmosphere for 28 days.

### <Example E-1c>

With the use of the Compound (12-6) obtained in Example S-1, an organic TFT was produced by forming an organic semiconductor layer on a "PVP substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-6) to chloroform such that the concentration was 0.1 wt% was dropped onto the "PVP substrate" under an atmosphere of nitrogen, and then spin-coating was performed at 1000 rpm for 30 seconds, to form an organic semiconductor layer with a thickness of about 20 nm. And then, the "PVP substrate" on which the organic semiconductor layer was formed was heated at 120°C for 35 minutes under an atmosphere of nitrogen.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-6) on the "PVP substrate" had a field-effect mobility of 2.9×10⁻¹ cm²/Vs.

### <Example E-1d>

With the use of the Compound (12-6) obtained in Example S-1, an organic TFT was produced by forming an organic semiconductor layer on a "bare substrate" by spin-coating in the atmosphere, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-6) to chloroform such that the concentration was 0.1 wt% was dropped onto the "bare substrate" in the atmosphere, and then spin-coating was performed at 1000 rpm for 30 seconds, to form an organic semiconductor layer with a thickness of about 20 nm. And then, the "bare substrate" on which the organic semiconductor layer was formed was heated at 120°C for 35 minutes in the atmosphere.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-6) on the "bare substrate" had a field-effect mobility of 3.2×10⁻¹ cm²/Vs.

### <Example E-1e>

With the use of the Compound (12-6) obtained in Example S-1, an organic TFT was produced by forming an organic semiconductor layer on a "PVP substrate" by spin-coating in the atmosphere, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-6) to chloroform such that the concentration was 0.1 wt% was dropped onto the "PVP substrate" in the atmosphere, and then spin-coating was performed at 1000 rpm for 30 seconds, to form an organic semiconductor layer with a thickness of about 20 nm. And then, the "PVP substrate" on which the organic semiconductor layer was formed was heated at 180°C for 35 minutes in the atmosphere.

### (Evaluation of organic TFT)

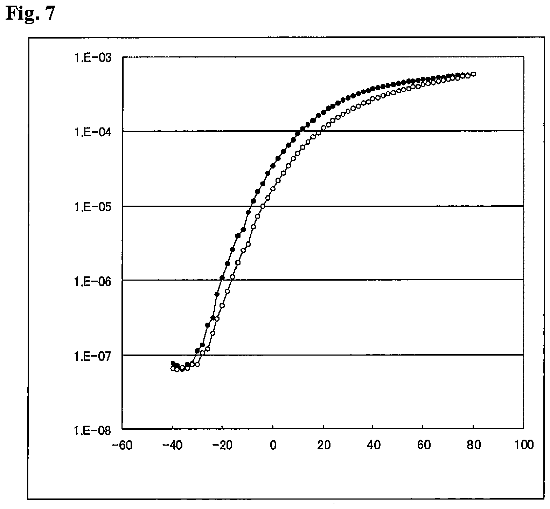

The transfer characteristics of the produced organic TFT were measured under the condition that the drain voltage was 80 V, and the organic TFT had the n-type semiconductor characteristics, while the organic TFT had not the p-type semiconductor characteristics. The obtained transfer characteristics are shown in Fig. 7. In Fig. 7, the horizontal axis indicates gate voltage (V), and the vertical axis indicates drain current (A). The curve with gray-lacquered circle indicates fore, and the curve with outline character circle indicates back.

The field-effect mobility (µ) was calculated using the above-described (Formula A), and as the result thereof, it was found that the organic TFT comprising Compound (12-6) on the "PVP substrate" had a field-effect mobility of 6.3×10⁻¹ cm²/Vs.

### <Example E-2a>

With the use of the Compound (22-1) obtained in Example S-3, an organic TFT was produced by forming an organic semiconductor layer on a "HMDS-modified substrate" by vacuum deposition, and then forming a source electrode and a drain electrode on the organic semiconductor layer as mentioned above, and was evaluated.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (22-1) on the "HMDS-modified substrate" had a field-effect mobility of 3.6×10⁻⁴ cm²/Vs.

### <Example E-2b>

With the use of the Compound (22-1) obtained in Example S-3, an organic TFT was produced by forming an organic semiconductor layer on a "PS substrate" by vacuum deposition, and then forming a source electrode and a drain electrode on the organic semiconductor layer as mentioned above, and was evaluated.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (22-1) on the "PS substrate" had a field-effect mobility of 5.2×10⁻⁴ cm²/Vs.

### <Example E-2c>

With the use of the Compound (22-1) obtained in Example S-3, an organic TFT was produced by forming an organic semiconductor layer on a "PVP substrate" by vacuum deposition, and then forming a source electrode and a drain electrode on the organic semiconductor layer as mentioned above, and was evaluated.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (22-1) on the "PVP substrate" had a field-effect mobility of 3.6×10⁻⁴ cm²/Vs.

### <Example E-3a>

With the use of the Compound (12-8) obtained in Example S-4, an organic TFT was produced by forming an organic semiconductor layer on a "bare substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-8) to chloroform such that the concentration was 0.1 wt% was dropped onto the "bare substrate" under an atmosphere of nitrogen, and then spin-coating was performed at 1000 rpm for 30 seconds, to form an organic semiconductor layer with a thickness of about 20 nm. And then, the "bare substrate" on which the organic semiconductor layer was formed was heated at 180°C for 35 minutes under an atmosphere of nitrogen.

### (Evaluation of organic TFT)

The transfer characteristics of the produced organic TFT were measured under the condition that the drain voltage was 80 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-8) on the "bare substrate" had a field-effect mobility of 1.0×10⁰ cm²/Vs.

### <Example E-3b>

With the use of the Compound (12-8) obtained in Example S-4, an organic TFT was produced by forming an organic semiconductor layer on a "PVP substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-8) to chloroform such that the concentration was 0.1 wt% was dropped onto the "PVP substrate" under an atmosphere of nitrogen, and then spin-coating was performed at 1000 rpm for 30 seconds, to form an organic semiconductor layer with a thickness of about 20 nm. And then, the "PVP substrate" on which the organic semiconductor layer was formed was heated at 180°C for 35 minutes under an atmosphere of nitrogen.

### (Evaluation of organic TFT)

The transfer characteristics of the produced organic TFT were measured under the condition that the drain voltage was 80 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-8) on the "PVP substrate" had a field-effect mobility of 9.6×10⁻¹ cm²/Vs.

### <Example E-3c>

With the use of the Compound (12-8) obtained in Example S-4, an organic TFT was produced by forming an organic semiconductor layer on a "bare substrate" by spin-coating in the atmosphere, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-8) to chloroform such that the concentration was 0.1 wt% was dropped onto the "bare substrate" in the atmosphere, and then spin-coating was performed at 1000 rpm for 30 seconds, to form an organic semiconductor layer with a thickness of about 20 nm. And then, the "bare substrate" on which the organic semiconductor layer was formed was heated at 180°C for 35 minutes in the atmosphere.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-8) on the "bare substrate" had a field-effect mobility of 8.3×10⁻¹ cm²/Vs.

### <Example E-3d>

With the use of the Compound (12-8) obtained in Example S-4, an organic TFT was produced by forming an organic semiconductor layer on a "PVP substrate" by spin-coating in the atmosphere, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-8) to chloroform such that the concentration was 0.1 wt% was dropped onto the "PVP substrate" in the atmosphere, and then spin-coating was performed at 1000 rpm for 30 seconds, to form an organic semiconductor layer with a thickness of about 20 nm. And then, the "PVP substrate" on which the organic semiconductor layer was formed was heated at 180°C for 35 minutes in the atmosphere.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-8) on the "PVP substrate" had a field-effect mobility of 5.2×10⁻¹ cm²/Vs.

### <Example E-4a>

With the use of the Compound (12-EH) obtained in Example S-5, an organic TFT was produced by forming an organic semiconductor layer on a "bare substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-EH) to chloroform such that the concentration was 0.1 wt% was dropped onto the "bare substrate" under an atmosphere of nitrogen, and then spin-coating was performed at 1000 rpm for 30 seconds, to form an organic semiconductor layer with a thickness of about 20 nm. And then, the "bare substrate" on which the organic semiconductor layer was formed was heated at 180°C for 35 minutes under an atmosphere of nitrogen.

### (Evaluation of organic TFT)

The transfer characteristics of the produced organic TFT were measured under the condition that the drain voltage was 80 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-EH) on the "bare substrate" had a field-effect mobility of 5.7×10⁻¹ cm²/Vs.

### <Example E-4b>

With the use of the Compound (12-EH) obtained in Example S-5, an organic TFT was produced by forming an organic semiconductor layer on a "PVP substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-EH) to chloroform such that the concentration was 0.1 wt% was dropped onto the "PVP substrate" under an atmosphere of nitrogen, and then spin-coating was performed at 1000 rpm for 30 seconds, to form an organic semiconductor layer with a thickness of about 20 nm. And then, the "PVP substrate" on which the organic semiconductor layer was formed was heated at 180°C for 35 minutes under an atmosphere of nitrogen.

### (Evaluation of organic TFT)

The transfer characteristics of the produced organic TFT were measured under the condition that the drain voltage was 80 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-EH) on the "PVP substrate" had a field-effect mobility of 2.0×10⁻¹ cm²/Vs.

### <Example E-5a>

With the use of the Compound (12-12) obtained in Example S-6, an organic TFT was produced by forming an organic semiconductor layer on a "bare substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-12) to chloroform such that the concentration was 0.1 wt% was dropped onto the "bare substrate" under an atmosphere of nitrogen, and then spin-coating was performed at 1000 rpm for 30 seconds, to form an organic semiconductor layer with a thickness of about 20 nm. And then, the "bare substrate" on which the organic semiconductor layer was formed was heated at 180°C for 35 minutes under an atmosphere of nitrogen.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-12) on the "bare substrate" had a field-effect mobility of 7.3×10⁻¹ cm²/Vs.

### (Evaluation of organic TFT)

Moreover, the transfer characteristics of the produced organic TFT were measured under the condition that the drain voltage was 20 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-12) on the "bare substrate" had a field-effect mobility of 3.3×10⁻¹ cm²/Vs.

### <Example E-5b>

With the use of the Compound (12-12) obtained in Example S-6, an organic TFT was produced by forming an organic semiconductor layer on a "PVP substrate" by spin-coating under an atmosphere of nitrogen, and then forming a source electrode and a drain electrode as mentioned above, and was evaluated. The condition of production of the organic semiconductor layer is as described below.

### (Conditions of production of organic semiconductor layer)

0.18 mL of a solution (organic semiconductor ink) which was prepared at room temperature by adding Compound (12-12) to chloroform such that the concentration was 0.1 wt% was dropped onto the "PVP substrate" under an atmosphere of nitrogen, and then spin-coating was performed at 1000 rpm for 30 seconds, to form an organic semiconductor layer with a thickness of about 20 nm. And then, the "PVP substrate" on which the organic semiconductor layer was formed was heated at 150°C for 35 minutes under an atmosphere of nitrogen.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-12) on the "PVP substrate" had a field-effect mobility of 6.2×10⁻¹ cm²/Vs.

### (Evaluation of organic TFT)

Moreover, the transfer characteristics of the produced organic TFT were measured under the condition that the drain voltage was 20 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-12) on the "PVP substrate" had a field-effect mobility of 3.5×10⁻¹ cm²/Vs.

### <Example E-6>

With the use of the Compound (12-HepFBu) obtained in Example S-7, an organic TFT was produced by forming an organic semiconductor layer on a "OTS-modified substrate" by vacuum deposition, and then forming a source electrode and a drain electrode on the organic semiconductor layer as mentioned above, and was evaluated.

### (Evaluation of organic TFT)

The transfer characteristics of the produced organic TFT were measured under the condition that the drain voltage was 100 V, and the organic TFT had the n-type semiconductor characteristics, while the organic TFT had not the p-type semiconductor characteristics.

The field-effect mobility (µ) was calculated using the above-described (Formula A), and as the result thereof, it was found that the organic TFT comprising Compound (12-HepFBu) on the "OTS-modified substrate" had a field-effect mobility of 6.4×10⁻³ cm²/Vs.

As can be seen from the above-described results, the benzobis(thiadiazole) derivative of the present invention has a high field-effect mobility.

### Industrial Applicability

According to the present invention, there may be provided a benzobis(thiadiazole) derivative which has an excellent mobility of electron (field-effect mobility), and also has an excellent stability in the atmosphere and which is generally soluble in an organic solvent and allows the formation of a thin film by a coating method. In particular, although it is known that the conventional n-type organic semiconductor materials which are soluble in organic solvents are dissolved in halogenated aromatic hydrocarbons and halogenated aliphatic hydrocarbons only, the benzobis(thiadiazole) derivative of the present invention may be soluble in aromatic hydrocarbons.

Because the benzobis(thiadiazole) derivative of the present invention is thermally stable and has a high field-effect mobility, the high field-effect mobility property may be achieved when the compound is used for a semiconductor layer of an organic TFT. In addition, high luminous efficiency may be achieved when the compound is used for, in particular, an electron transport layer of an organic EL device. Additionally, high photoelectric conversion efficiency may be achieved when the compound is used for a charge separation layer and/or an electron transport layer of an organic thin film photovoltaic cell.

In addition, the display device comprising arranged pixels, in which the organic TFT of the present invention, and the organic EL device of the present invention or other type of organic EL device are combined, has the advantages of having an excellent luminous efficiency; and having excellent response properties. Additionally, the organic TFT of the present invention may be suitably used as an organic TFT by which a RFID tag or a sensor is activated.

### Description of the Main Symbols:

11, 21, 31, 111 Substrate
12, 106 Gate electrode
13, 107 Gate insulating layer
14, 110 Drain electrode
15, 109 Source electrode
16, 108 Organic semiconductor layer
22, 105 Anode
23, 104 Hole transport layer
24, 103 Luminescent layer
25, 102 Electron transport layer
26, 101 Cathode
112 Barrier layer
113 Protective layer
120 Organic EL device
121 Organic TFT
32 Anode
33 Charge separation layer
34 Cathode

## Claims

1. A benzobis(thiadiazole) derivative, which has cyclic imide structures annelated to an aromatic ring in the molecule, represented by the following general formula (1) or (2): 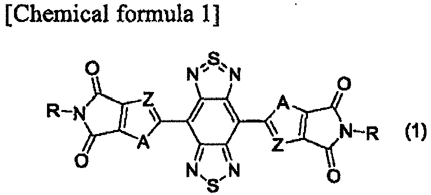 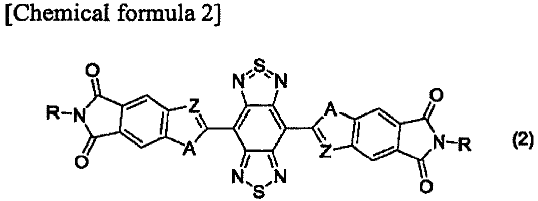 Wherein the above formulae (1) and (2),
two Rs represent independently a linear or branched alkyl group, or a linear or branched arylalkyl group, wherein a hydrogen atom in the alkyl group and in the alkyl group the in arylalkyl group can be substituted by a fluorine atom,
two As represent independently an oxygen atom, a sulfur atom or a selenium atom, and
two Zs represent independently a methine carbon or a nitrogen atom.

2. The benzobis(thiadiazole) derivative according to Claim 1, wherein two As are a sulfur atom, two Zs are independently a methine carbon or a nitrogen atom, and two Rs are independently a linear or branched alkyl group, wherein a hydrogen atom in the alkyl group can be substituted by a fluorine atom.

3. The benzobis(thiadiazole) derivative according to Claim 1 or 2, wherein two Rs are a linear or branched alkyl group of 5 to 25 carbon atoms wherein a hydrogen atom in the alkyl group can be substituted by a fluorine atom.

4. The benzobis(thiadiazole) derivative according to any one of Claims 1 to 3, wherein the benzobis(thiadiazole) derivative is a compound represented by the general formula (1).

5. The benzobis(thiadiazole) derivative according to any one of Claims 1 to 4, wherein the benzobis(thiadiazole) derivative is soluble in an organic solvent.

6. An organic semiconductor ink comprising the benzobis(thiadiazole) derivative according to any one of Claims 1 to 5.

7. An organic semiconductor ink comprising two or more of organic semiconductors, wherein one or more of the organic semiconductors are the benzobis(thiadiazole) derivative according to any one of claims 1 to 5.

8. An organic electronic device comprising an organic layer, which comprises the benzobis(thiadiazole) derivative according to any one of Claims 1 to 5.

9. An organic thin film transistor, comprising a gate electrode, a gate insulating layer, an organic semiconductor layer, a source electrode, and a drain electrode on a substrate, wherein the organic semiconductor layer comprises the benzobis(thiadiazole) derivative according to any one of Claims 1 to 5.

10. The organic thin film transistor according to Claim 9, wherein the substrate is a flexible substrate.

11. An organic electroluminescence device, comprising an anode, a luminescent layer, a hole transport layer and/or an electron transport layer, a cathode on a substrate, wherein the hole transport layer and/or the electron transport layer comprise the benzobis(thiadiazole) derivative according to any one of Claims 1 to 5.

12. The organic electroluminescence device according to Claim 11, wherein the substrate is a flexible substrate.

13. A display device, in which an organic electroluminescence device is driven/lighted by the use of an organic thin film transistor, wherein the organic thin film transistor is the organic thin film transistor according to Claim 9 or 10.

14. An active-matrix display device, wherein pixels are arranged in a matrix form, the pixel comprising the organic thin film transistor according to Claim 9 or 10 and an organic electroluminescence device.

15. The display device according to Claim 13 or 14, wherein the organic electroluminescence device is the organic electroluminescence device according to Claim 11 or 12.

16. A display device, in which an organic electroluminescence device is driven/lighted by the use of an organic thin film transistor, wherein the organic electroluminescence device is the organic electroluminescence device according to Claim 11 or 12.

17. An organic thin film photovoltaic cell, comprising a cathode, a charge separation layer comprising a hole transport material and an electron transport material, and an anode on a substrate, wherein the charge separation layer comprises the benzobis(thiadiazole) derivative according to any one of Claims 1 to 5.

18. An organic thin film photovoltaic cell, comprising an anode, a charge separation layer comprising a hole transport material and an electron transport material, a hole transport layer and/or an electron transport layer, and a cathode on a substrate, wherein the hole transport layer and/or the electron transport layer comprise the benzobis(thiadiazole) derivative according to any one of Claims 1 to 5.

19. The organic thin film photovoltaic cell according to Claim 17 or 18, wherein the substrate is a flexible substrate.

20. A RFID tag, which is activated by the use of an organic thin film transistor, wherein the organic thin film transistor is the organic thin film transistor according to Claim 9 or 10.

21. A sensor, which is activated by the use of an organic thin film transistor, wherein the organic thin film transistor is the organic thin film transistor according to Claim 9 or 10.
